# EUROPEAN PATENT APPLICATION

(11) **EP 4 043 457 A1**
(43) Date of publication of application: **17.08.2022**
(21) Application number: 20863959.1
(22) Date of filing: 09.09.2020
(51) Int. Cl.: C07D 413/14, C07D 405/14, C07D 487/10, C07D 409/14, A61K 31/4439, A61K 31/4545, A61P 35/00, A61P 37/02

(54) **AROMATIC HETEROCYCLIC COMPOUND HAVING TRICYCLIC STRUCTURE, AND PREPARATION METHOD THEREFOR AND APPLICATION THEREOF**

(30) Priority: 09.09.2019 CN 201910848895
(71) Applicant: Shanghai Longwood Biopharmaceuticals Co., Ltd., Shanghai 201108 (CN)
(72) Inventor: WANG, Zhe, Shanghai 201108 (CN); BAI, Haiyun, Shanghai 201108 (CN); LI, Deliang, Shanghai 201108 (CN); QIAN, Anran, Shanghai 201108 (CN)
(74) Representative: Lavoix
(86) International application number: PCT/CN2020/114261
(87) International publication number: WO 2021/047553

(57) **Abstract**

An aromatic heterocyclic compound having a tricyclic structure and a preparation method therefor and application thereof, and in particular, a compound as shown in formula I, or an optical isomer, a hydrate, a solvate, or a pharmaceutically acceptable salt thereof. The compound shown in formula I may be used to treat diseases associated with a PD-1/PD-L1 signaling pathway.

## Description

### TECHNICAL FIELD

The present invention relates to the field of small molecule drugs, and specifically, the present invention provides a small molecule compound that can be used to treat diseases related to PD-1/PD-L1 signaling pathway.

### BACKGROUND OF THE INVENTION

The immune system plays a vital role in controlling and curing many diseases, such as various cancers, diseases caused by viruses, etc. But the cancer cells can evade or modulate the immune system in some way to proliferate rapidly. One way is to alter the activator molecules and inhibitory molecules expressed on immune cells. Blocking regulatory immune checkpoints, such as PD-1, has been proven to be a very effective approach to suppressing cancer cells.

PD-1 is programmed cell death protein-1, also known as CD279. It is mainly expressed in activated T cells and B cells, and its function is to regulate the activation of cells, which is a normal homeostatic mechanism of the immune system, because excessive T/B cell activation can cause autoimmune diseases, PD-1 is a protective wall of our body. PD-1 is a type I transmembrane glycoprotein composed of 268 amino acids, and its structure mainly includes the outer immunoglobulin variable domain, the hydrophobic transmembrane domain and the intracellular domain. The intracellular domain contains two phosphorylation sites, and they are located in the immunoreceptor tyrosine regulatory motif and the immunoreceptor tyrosine switching motif, respectively, which also proves that PD-1 can negatively regulate T cell receptor-mediated signal. PD-1 has two ligands, PD-L1 and PD-L2, which differ in expression way. PD-L1 is up-regulated in a variety of tumor cells, and it binds to PD-1 on T cells, regulates T cell proliferation and activation, makes T cells in a state of inactivation, and ultimately induces immune escape.

PD-1/PD-L1 plays an inverse immunomodulatory role. When PD-1 binds to PD-L1, it can cause tyrosine polyphosphorylation in the immunoreceptor tyrosine switch motif domain of T cells, and the phosphorylated tyrosine can bind to the phosphatase protein tyrosinase 2 and protein tyrosinase 1, which can impede the activation of extracellular signal-regulated kinase and also block the activation of phosphatidylinositol 3-kinase (PI3K) and serine-threonine protein kinase (Akt), thereby regulating T lymphocyte proliferation and the secretion of related cytokines. The PD-1/PD-L1 signal inhibits the activation and proliferation of T cells, and at the same time, it can also induce the secretion of cytokines interleukin 2, interferon γ and IL-10. In addition, PD-1/PD-L1 signaling also has a similar immune function on B cells. After PD-1 binds to B cell antigen receptors, the PD-1 cytoplasmic domain interacts with tyrosinase containing a protein tyrosinase 2 binding site, thereby hindering B cell activation.

The immunotherapy based on PD-1/PD-L1 is a new generation of immunotherapy that has attracted much attention. In recent years, a series of surprising findings have confirmed that PD-1/PD-L1 modulators have strong antitumor activity against a variety of tumors. Currently available PD-1/PD-L1 antibody inhibitors include BMS's Ninolumab, Merck's Lambrolizumab and Roche's Atezolizumab. In addition, there are many PD-1/PD-L1 antibody modulators in development, including CureTech's Pidilizumab, GSK's AMP-224 and AstraZeneca's MEDI-4736.

Although tumor immunotherapy is considered to be a new generation of revolution in cancer treatment after targeted therapy, the PD-1 monoclonal antibodies currently on the market and under development have their own shortcomings. They can only be administered by injection and cannot be taken orally. They are unstable in the body, are easily decomposed by proteases, and are prone to immune cross-reaction. Moreover, it is difficult to purify and the production cost is high. Therefore, small molecule modulator of PD-1/PD-L1 interaction is a better option for tumor immunotherapy or viral infection therapy.

In summary, there is an urgent need in the art to develop novel small-molecule modulator of PD-1/PD-L1 interaction.

### SUMMARY OF THE INVENTION

The object of the present invention is to provide a novel small-molecule modulator of PD-1/PD-L1 interaction.

The first aspect of the present invention provides a compound represented by the following formula I, or an optical isomer, a hydrate, a solvate thereof, or a pharmaceutically acceptable salt thereof: 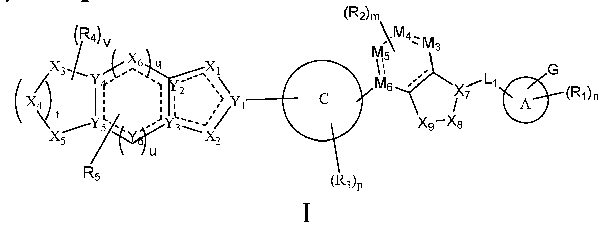 wherein, n, q, m, t, p, v, and u are each independently 0, 1, 2, 3, 4, or 5;
X₁, X₂, X₃, X₄, X₅ and X₆ are each independently selected from the group consisting of N, O, S, SO, SO₂, C(R)₂, CHR, and NR;
Y₁, Y₂, Y₃, Y₄, Y₅ and Y₆ are each independently selected from the group consisting of N, CH, and C;
X₇ is selected from the group consisting of N, and CR;
X₈ and X₉ are each independently selected from the group consisting of O, S, SO, SO₂, C(R)₂, NR; wherein, R is selected from the group consisting of H, C₁-C₆ alkyl, chlorine, bromine, fluorine, iodine, cyano, hydroxy, nitro, NRf, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NRdRe, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, -C(=O)-substituted or unsubstituted C₂-C₆ alkynyl; wherein, a carbon atom of X₇, X₈, X₉ may be each independently replaced by deuterium;
M₃, M₄, M₅ are each independently selected from the group consisting of: chemical bond, CR, N, NR, O, S, SO, and SO₂;
M₆ is each independently selected from the group consisting of CR, N, and C; and 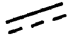 represents single bond or double bond; 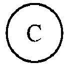 is each independently selected from the group consisting of substituted or unsubstituted C5-C12 arylene, substituted or unsubstituted 5-12 membered (preferably 5-7 membered) heteroarylene having 1-3 heteroatoms, substituted or unsubstituted 5-12 membered heterocyclylene, substituted or unsubstituted C5-C12 cycloalkylene; 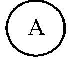 is selected from the group consisting of substituted or unsubstituted 5-12 membered heteroaryl, substituted or unsubstituted C6-C10 aryl, substituted or unsubstituted 3-12 (preferably 5-12) membered heterocyclyl, substituted or unsubstituted C3-C12 (preferably C5-C12) cycloalkyl, wherein the heterocyclyl has 1-3 heteroatoms;
L₁ is selected from the group consisting of chemical bond, substituted or unsubstituted C1-C4 alkylene, substituted or unsubstituted C2-C4 alkenylene, substituted or unsubstituted C2-C4 alkynylene, -S-, -O-, substituted or unsubstituted -NH-, -S(O)-, -S(O)₂-, substituted or unsubstituted -NHC(O)NH-, 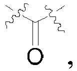 substituted or unsubstituted 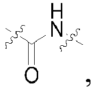 substituted or unsubstituted 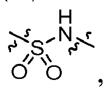 substituted or unsubstituted 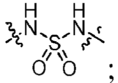
G is absent, H, substituted or unsubstituted C1-C6 alkyl, or 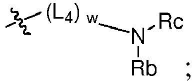 wherein, w is 0, 1, 2, 3, 4, 5, 6;
each L₄ is independently selected from the group consisting of substituted or unsubstituted C1-C4 alkylene, -S-, -O-, NR_{f}, -S(O)-, -S(O)₂-; preferably substituted or unsubstituted C1-C4 alkylene; wherein, a hydrogen on a carbon atom of the substituted or unsubstituted C1-C4 alkylene may be each independently replaced by deuterium, provided that a structure formed by each L₄ is chemically stable;
Rb and Rc are each independently selected from the group consisting of H, substituted or unsubstituted C₁-C₈ alkyl; or Rb and Rc together with the adjacent N atom form substituted or unsubstituted 5-10 3-10 membered heterocyclyl having 1-3 heteroatoms selected from N, S and O;
R₁, R₂, R₃, R₄, R₅, G and R are each independently selected from the group consisting of H, -CN, trifluoromethyl, -CHF₂, -OCF₃, -OCHF₂, sulfonamido, nitro, hydroxyl, halogen, -S-R₈, -S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted -(C1-C6 alkylene)-NH-(C1-C6 alkylene), carboxy, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, wherein a hydrogen on a carbon atoms of substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e. =O), =NR_{f}, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms can be each independently replaced by deuterium; substituted or unsubstituted 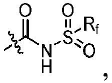 substituted or unsubstituted 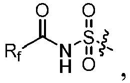 substituted or unsubstituted 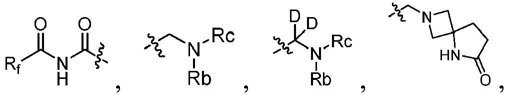 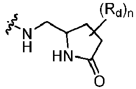 (please modify the structure here), 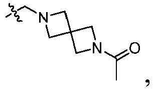 wherein, Rb, Rc and Rd are are each independently selected from the group consisting of H, substituted or unsubstituted C₁-C₈ alkyl; or Rb and Rc together with adjacent N atom form substituted or unsubstituted 3-10 membered heterocyclyl having 1-3 heteroatoms selected from N, S and O, or Rb and Rc together with adjacent N atom form substituted or unsubstituted 4-10 membered ring; wherein, a hydrogens on a carbon atom of Rb, Rc and Rd may be each independently replaced by deuterium; or -(L₁ₐ)ᵣ-(L₂ₐ)ₛ-(L3a)ₛ-; -C₀₋₈-O-R₈, -C₀₋₈-C(O)OR₈, -C₀₋₈-OC(O)OR₈, -C₀₋₈-NR₈R₉, - C₀₋₈-N(R₈)C(O)R₉, -C₀₋₈-C(O)NR₈R₉;
R₈ and R₉ are each independently selected from the group consisting of H, hydroxy, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted-(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 5-12-membered heterocyclyl with 1-4 heteroatoms, substituted or unsubstituted 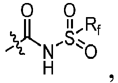 substituted or unsubstituted 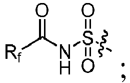 substituted or unsubstituted 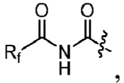 Or -(L₁ₐ)ᵣ-(L₂ₐ)ₛ-(L₃ₐ)ₛ-;
each L₁ₐ is each independently the group selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, or -S(O)₂-;
L₂ₐ is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, substituted or unsubstituted 5-10 membered heterocyclylene with 1-3 heteroatoms;
L₃ₐ is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g}, -OR_{g}, -N(R_{g})₂, -CO₂R_{g}, -CON(R_{g})₂, -CONHCOR_{g}, NR_{g}-CO-N(R_{g})₂, -NRg-SO₂-N(Rg)₂;
r is 1, 2, 3, 4, 5, 6;
s is 0, 1, 2;
Rd, Re and Rg are each independently selected from the group consisting of H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 3-10 (preferably 5-10) membered cycloalkyl, or 3-10 (preferably 5-10) membered heterocyclyl having 1-3 heteroatoms selected from N, S and O;
Rf is selected from the group consisting of H, substituted or unsubstituted C₁-C₆ alkyl, substituted or unsubstituted C₆-C₁₀ aryl, substituted or unsubstituted C₆-C₁₀ heteroaryl, cyano, -C(=O)-NRdRe, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, -C(=O)-substituted or unsubstituted C₂-C₆ alkynyl;
unless otherwise specified, the "substituted" refers to substitution with one or more (eg 2, 3, 4, etc.) substituents selected from the group consisting of halogen (including -F, Cl, Br), -CH₂Cl, -CHCl₂, -CCl₃, -CH₂F, -CHF₂, -CF₃; oxo (=O), - CN, hydroxyl, amino, C1-C6 alkylamino, carboxyl, -NHAc, 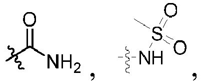 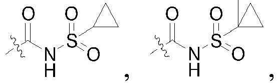 an unsubstituted or substituted group selected from the group consisting of C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, 5-10 membered heterocyclyl with 1-3 heteroatoms selected from N, S and O, which is substituted by one or more substituents selected from the following group; the substituent is selected from the group consisting of halogen, hydroxyl, carboxyl, cyano, C1-C6 alkoxy, C1-C6 alkylamino;
in the above formulas, any one of the heteroatoms is selected from the group consisting of B, P, N, S and O.

In another preferred embodiment, provided is a compound represented by the following formula I, or an optical isomer, a hydrate, a solvate thereof, or a pharmaceutically acceptable salt thereof: 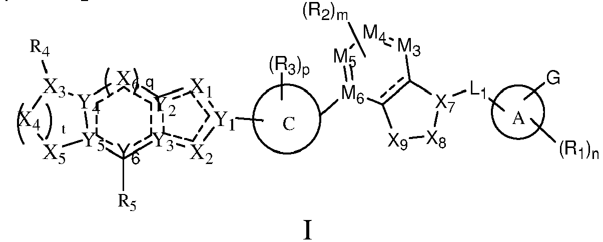 wherein, n, q, m, t, and p are each independently selected from 0, 1, 2, 3 or 4;
X₁, X₂, X₃, X₄, X₅ and X₆ are each independently selected from the group consisting of N, O, S, SO, SO₂, C(R)₂, NR;
Y₁, Y₂, Y₃, Y₄, Y₅ and Y₆ are each independently selected from the group consisting of N, CH, C;
X₇ is selected from the group consisting of N, CR;
X₈ and X₉ are each independently selected from the group consisting of O, S, SO, SO₂, C(R)₂, NR; wherein, R is selected from the group consisting of H, C₁-C₆ alkyl, chlorine, bromine, fluorine, iodine, cyano, hydroxy, nitro, NRf, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NRdRe, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, -C(=O)-substituted or unsubstituted C₂-C₆ alkynyl;
M₃, M₄, M₅ are each independently selected from the group consisting of: chemical bond, CR, N, NR, O, S, SO, SO₂;
M₆ is each independently selected from the group consisting of CR, N, C; and 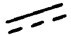 represents single bond or double bond; 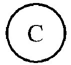 are each independently selected from the group consisting of substituted or unsubstituted C5-C12 arylene, or substituted or unsubstituted 5-12 membered (preferably 5-7 membered) heteroarylene having 1-3 heteroatoms, substituted or unsubstituted 5-12 membered heterocyclylene, substituted or unsubstituted C5-C12 cycloalkylene; 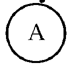
is selected from the group consisting of substituted or unsubstituted 5-12 membered heteroaryl, substituted or unsubstituted C6-C10 aryl, substituted or unsubstituted 5-12 membered heterocyclyl, substituted or unsubstituted C5-C12 cycloalkyl, wherein the heterocyclyl has 1-3 heteroatoms;
L₁ is selected from the group consisting of chemical bond, substituted or unsubstituted C1-C4 alkylene, substituted or unsubstituted C2-C4 alkenylene, substituted or unsubstituted C2-C4 alkynylene, -S-, -O-, substituted or unsubstituted -NH-, -S(O)-, -S(O)₂-, substituted or unsubstituted -NHC(O)NH-, 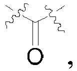 substituted or unsubstituted 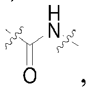 substituted or unsubstituted 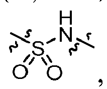 substituted or unsubstituted 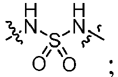
G is 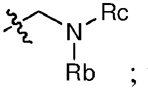 wherein, Rb and Rc are each independently selected from the group consisting of H, substituted or unsubstituted C₁-C₈ alkyl; or Rb and Rc together with the adjacent N atom form substituted or unsubstituted 5-10 membered heterocyclyl having 1-3 heteroatoms selected from N, S and O;
R₁, R₂, R₃, R₄, R₅, G and R are each independently selected from the group consisting of H, -CN, trifluoromethyl, sulfonamido, nitro, hydroxyl, halogen, -S-R₈, -S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted -(C1-C6 alkylene)-NH-(C1-C6 alkylene), carboxy, substituted or unsubstituted C6-C10 aryl, substituted or unsubstituted 5-12 membered heteroaryl with 1-3 heteroatoms, substituted or unsubstituted 5-12 membered heterocyclyl with 1-4 heteroatoms, substituted or unsubstituted 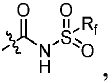 substituted or unsubstituted 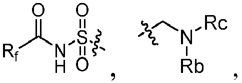 wherein, Rb and Rc are are each independently selected from the group consisting of H, substituted or unsubstituted C₁-C₈ alkyl; or Rb and Rc together with adjacent N atom form substituted or unsubstituted 3-10 membered heterocyclyl having 1-3 heteroatoms selected from N, S and O; or -(L₁ₐ)ᵣ-(L₂ₐ)ₛ-(L₃ₐ)ₛ-; -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉;
R₈ and R₉ are each independently selected from the group consisting of H, hydroxy, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted-(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 5-12 membered heterocyclyl with 1-4 heteroatoms, substituted or unsubstituted 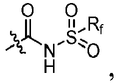 substituted or unsubstituted 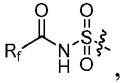 Or -(L₁ₐ)ᵣ-(L₂ₐ)ₛ-(L₃ₐ)ₛ-;
each L₁ₐ is each independently a group selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, and -S(O)₂-;
L₂ₐ is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, substituted or unsubstituted 5-10 membered heterocyclylene with 1-3 heteroatoms;
L₃ₐ is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g};
r is 1, 2, 3, 4, 5, 6;
s is 0, 1, 2 respectively;
Rd, Re and Rg are each independently selected from the group consisting of H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 5-10 membered cycloalkyl, or 5-10 membered heterocyclyl having 1-3 heteroatoms selected from N, S and O;
Rf is selected from the group consisting of H, substituted or unsubstituted C₁-C₆ alkyl, substituted or unsubstituted C₆-C₁₀ aryl, substituted or unsubstituted C₆-C₁₀ heteroaryl, cyano, -C(=O)-NRdRe, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, -C(=O)-substituted or unsubstituted C₂-C₆ alkynyl;
unless otherwise specified, the "substituted" refers to substitution with one or more (eg 2, 3, 4, etc.) substituents selected from the group consisting of halogen including but not limit to -F, Cl, Br, -CH₂Cl, -CHCl₂, -CCl₃, -CH₂F, -CHF₂, -CF₃, oxo, -CN, hydroxyl, amino, C1-C6 alkylamino, carboxyl, -NHAc, a group selected from the group consisting of C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, 5-10 membered heterocyclyl with 1-3 heteroatoms selected from N, S and O, which is unsubstituted or substituted by one or more substituents selected from the following group; the substituent is selected from the group consisting of halogen, hydroxyl, carboxyl, cyano, C1-C6 alkoxy, C1-C6 alkylamino;
in the above formulas, any one of the heteroatom are selected from the group consisting of B, P, N, S and O.

In another preferred embodiment, 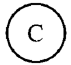 is benzene ring, preferably, R₃ is methyl, C1-C6 alkyl or alkoxy; C3-C6 cycloalkyl; C2-C6 alkalkenyl; C2-C6 alkynyl; halogen including -F, Cl, Br, -CH₂Cl, -CH₂Br, -CHCl₂, -CCl₃, -CH₂F, -CHF₂, -CF₃; - CN, hydroxyl, amino or alkyl substituted amino.

In another preferred embodiment, 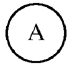 has a structure selected from the group consisting of 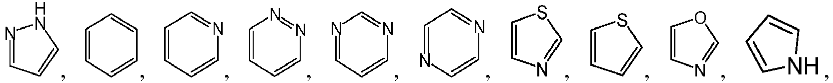 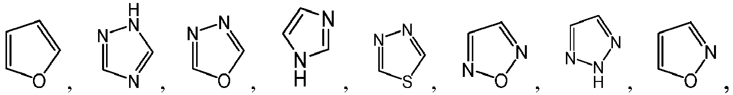 wherein the bonding position of the ring can be N or C (substituents on the ring are not shown).

In another preferred embodiment, 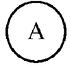 ring has a substitutent as shown in the following formula IV: 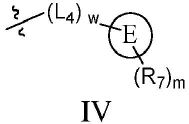 wherein, w is 0, 1, 2, 3, 4, 5, 6;
each L₄ is independently selected from the group consisting of substituted or unsubstituted C1-C4 alkylene, -S-, -O-, NR_{f}, -S(O)-, -S(O)₂-; preferably substituted or unsubstituted C1-C4 alkylene; wherein, a hydrogen on a carbon atom of the substituted or unsubstituted C1-C4 alkylene may be each independently replaced by deuterium, provided that a structure formed by each L₄ is chemically stable; 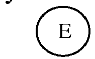
is selected from the group consisting of substituted or unsubstituted C3-C10 cycloalkyl, substituted or unsubstituted 3-10 membered heterocyclyl having 1-3 heteroatoms selected from B, P, N, S and O; preferably, 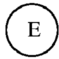 is 3-8 membered nitrogen-containing heterocyclyl, or substituted or unsubstituted 4-10 membered cyclic amido, wherein a hydrogen on the ring-forming carbon atom of 3-8 membered nitrogen-containing heterocyclyl, substituted or unsubstituted 4-10 membered cyclic amido may be independently replaced by deuterium;
each R₇ is independently selected from the group consisting of substituted or unsubstituted C1-C6 alkyl, -CN, hydroxy, amino, carboxyl, -OR_{g}, -N(R_{g})₂, -CO-NH-SO₂-R_{g}, -NH-SO₂-R_{g}, -SO₂-NH-CO-R_{g}, -CO₂R_{g}, -CON(R_{g})₂, -CONHCOR_{g}, NR_{g}-CO-N(R_{g})₂, -NRg-SO₂-N(Rg)₂;R_{f} and R_{g} are defiend as above; wherein a hydrogen on a carbon atom of Rf and Rg may be independently replaced by deuterium; wherein, the substituent is selected from the group consisting of halogen, hydroxyl, carboxyl, cyano, and C1- C6 alkoxy.

In another preferred embodiment, the compound of formula I has a structure shown in the following formula:

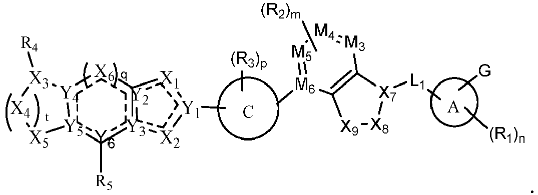

In another preferred embodiment, the compound of formula I has a structure shown in the following formula:

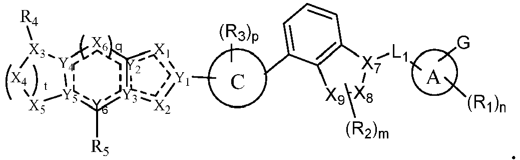

In another preferred embodiment, 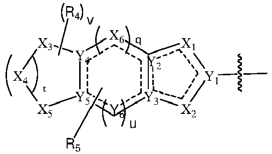 is selected from the group consisting of: 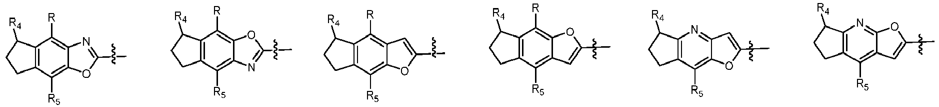 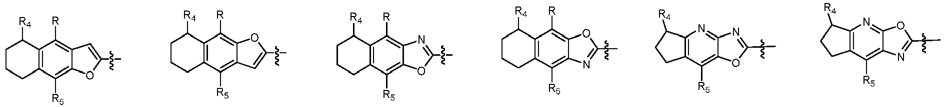 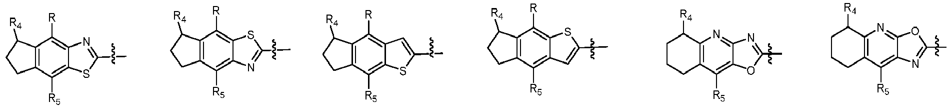 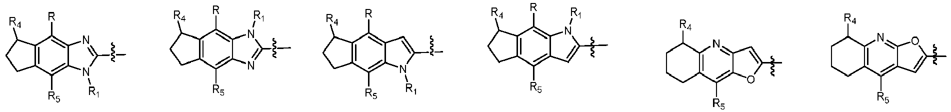 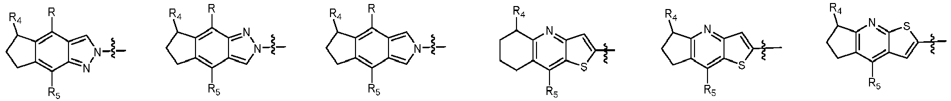 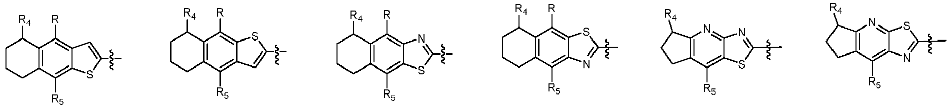 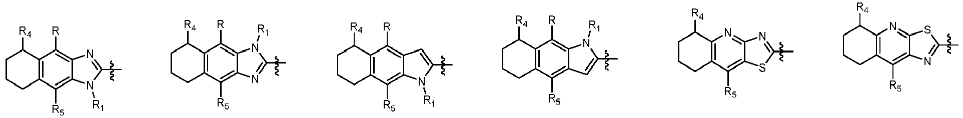 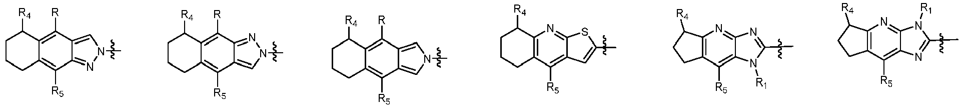 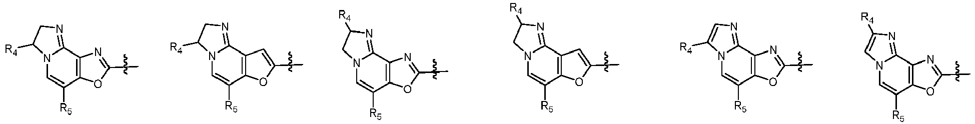 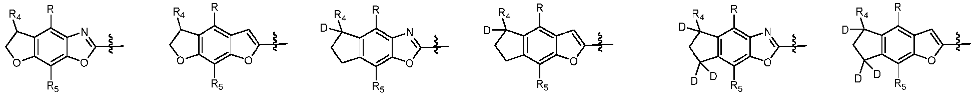 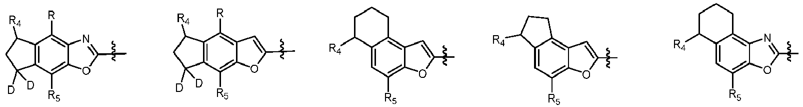 and

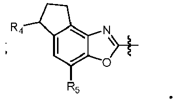

In another preferred embodiment, R₄ is selected from the group consisting of NRdRe; Rd and Re are each independently selected from the group consisting of H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 5-10 membered cycloalkyl, or 5-10 membered heterocyclyl having 1-3 heteroatoms selected from N, S and O; in another preferred embodiment, the substituent described here is carboxyl, hydroxyl, 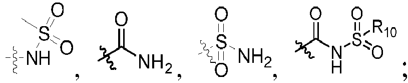 R₁₀ is substituted or unsubstituted C₁-C₆ alkyl, substituted or unsubstituted C₃-C₁₀ cycloalkyl, preferably, R₁₀ is methyl, isopropyl, cyclopropyl and 1-methylcyclopropyl, wherein a hydrogen on a carbon atom of Rd, Re and R₁₀ may be independently replaced by deuterium.

In another preferred embodiment, the compound is selected from the following table:

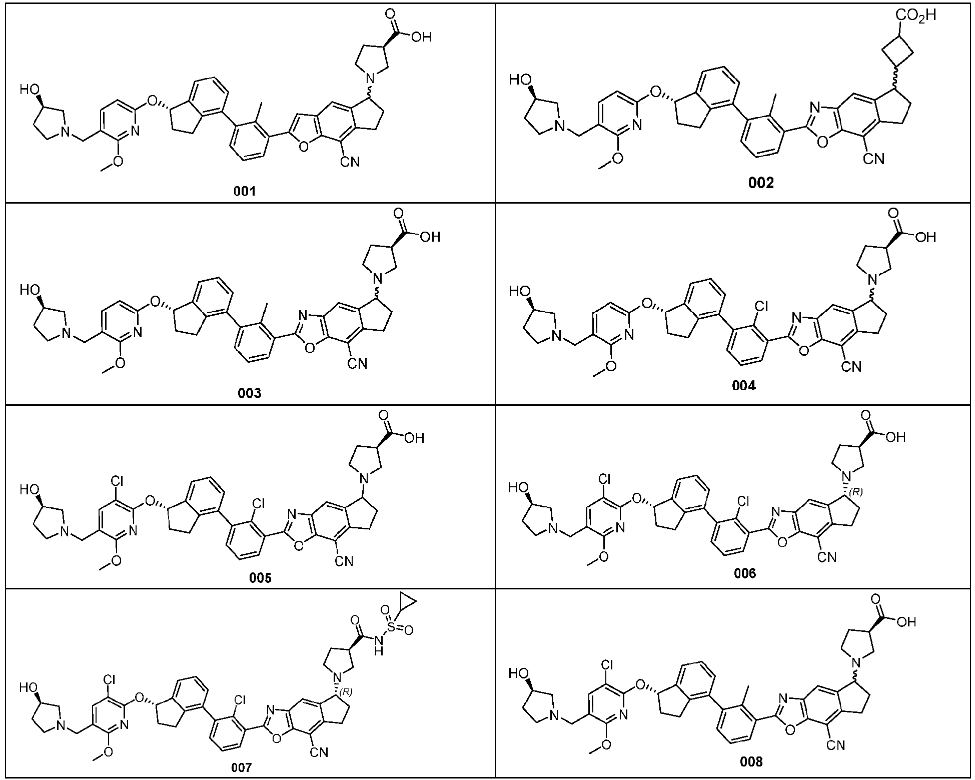

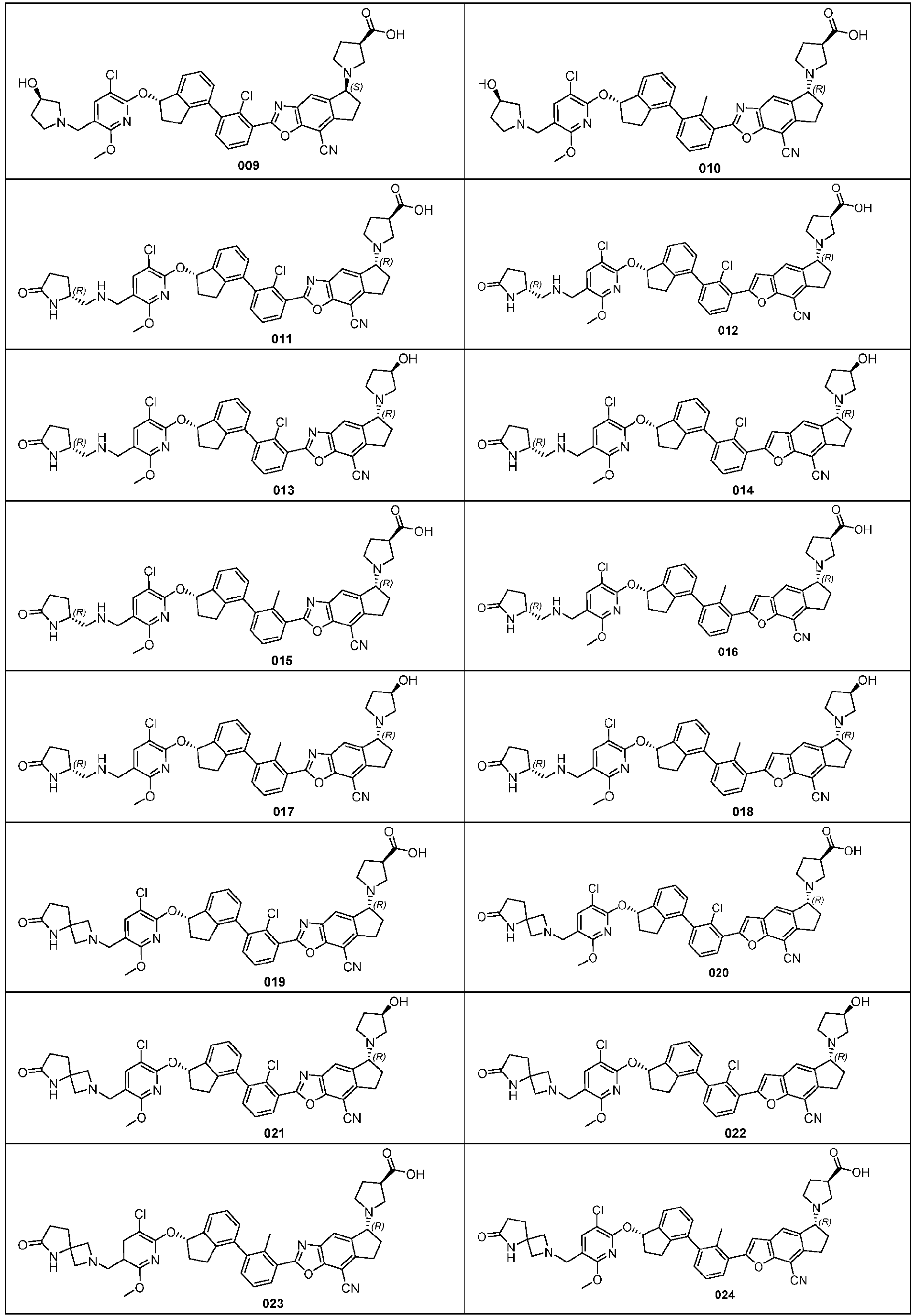

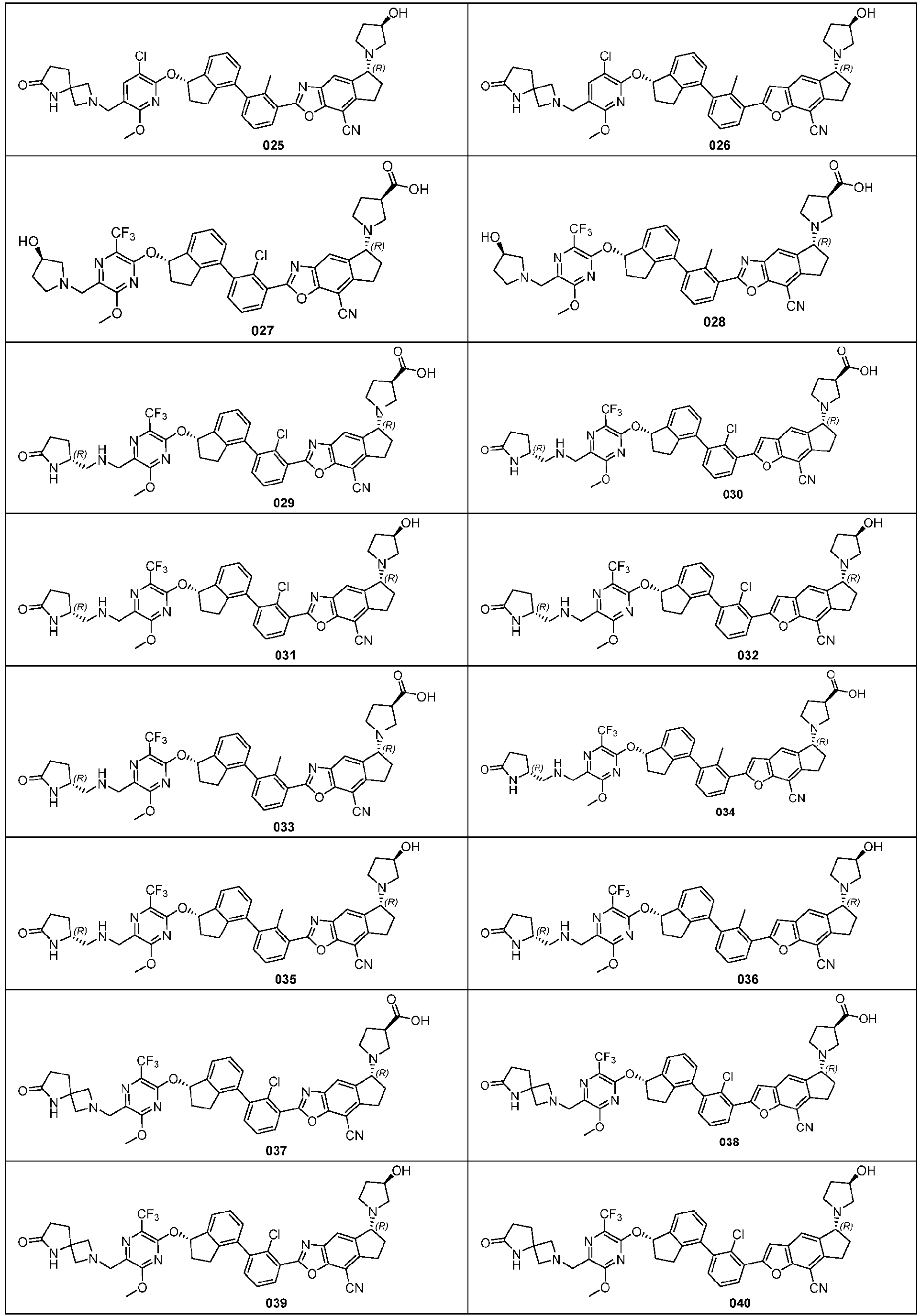

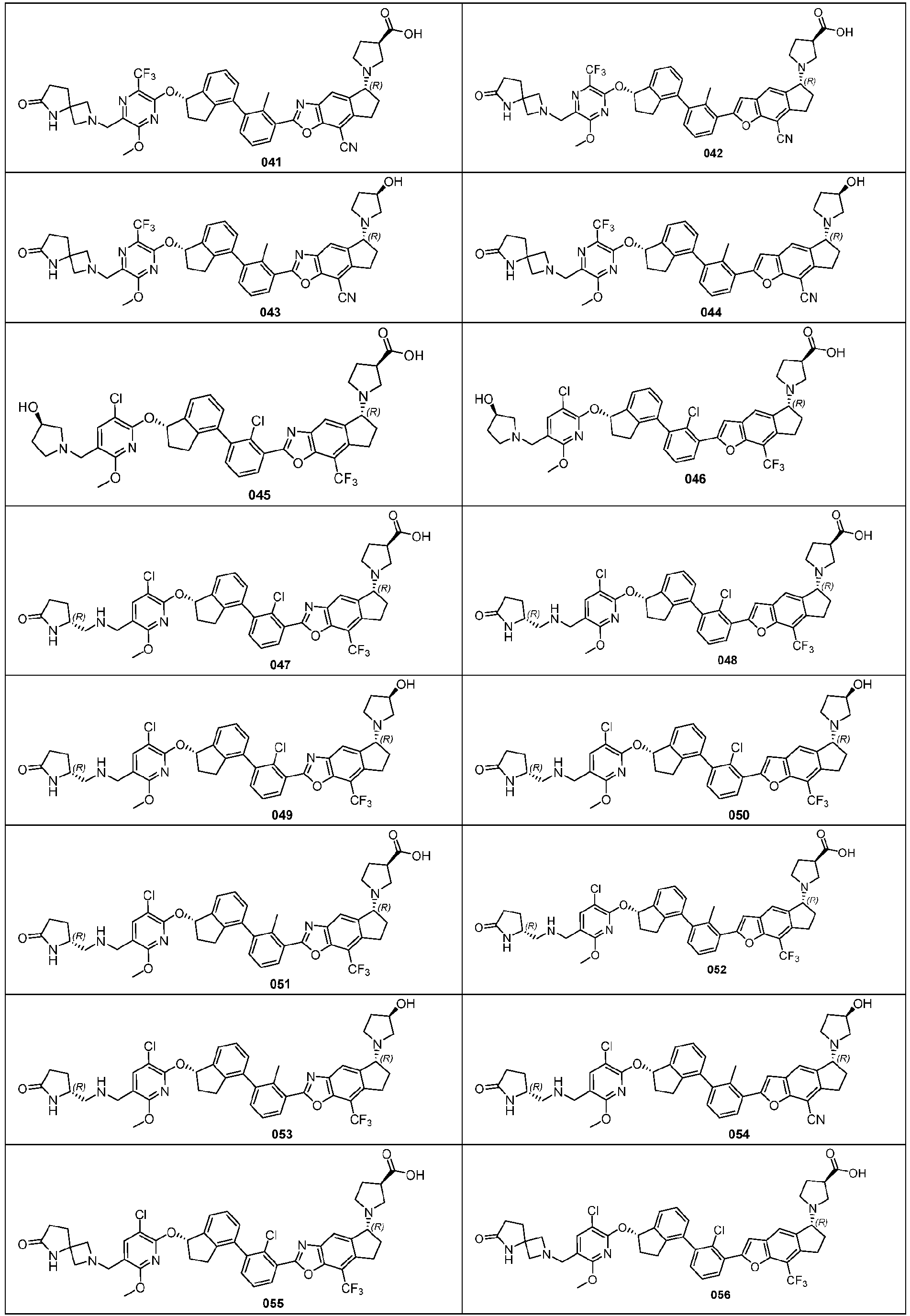

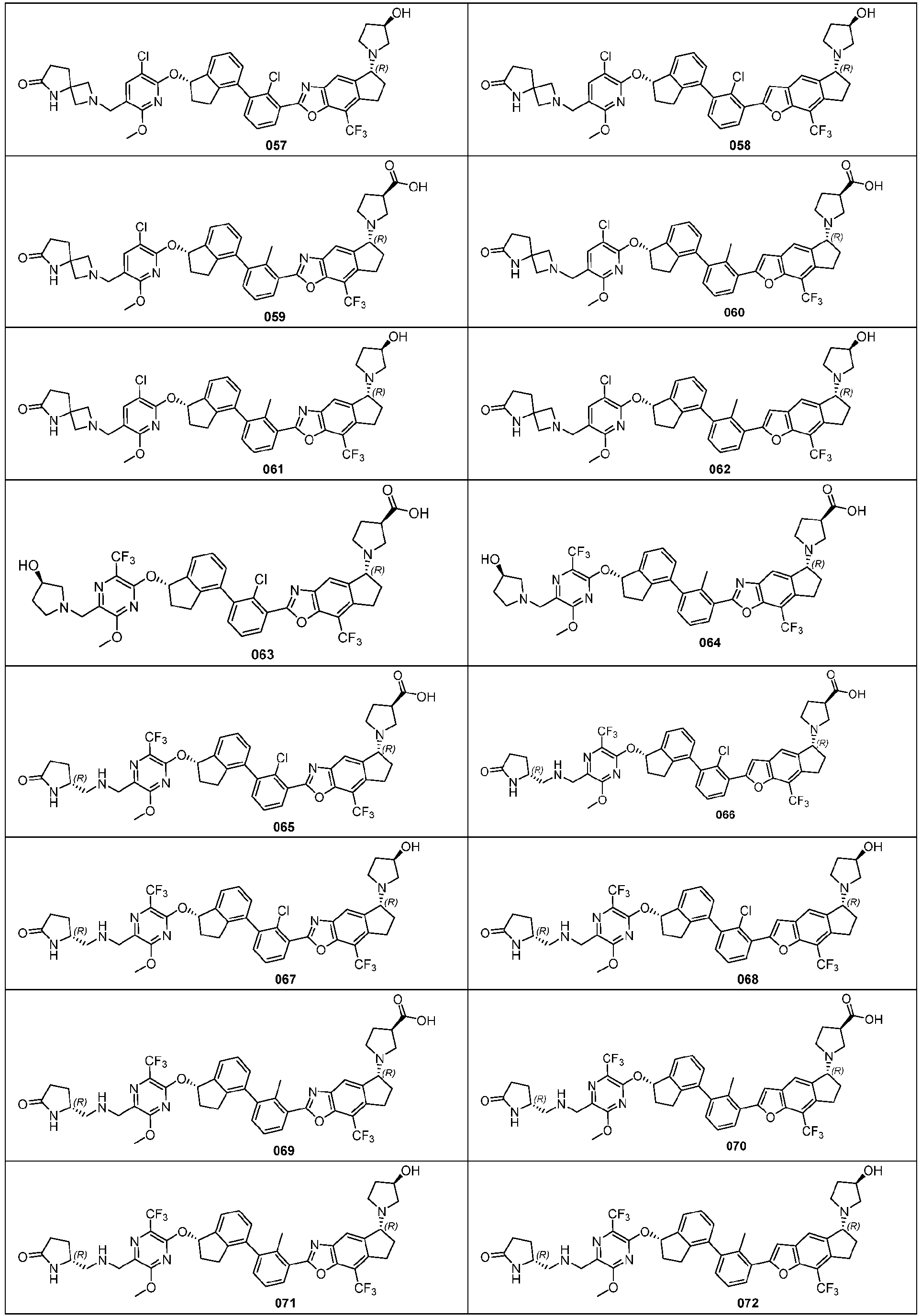

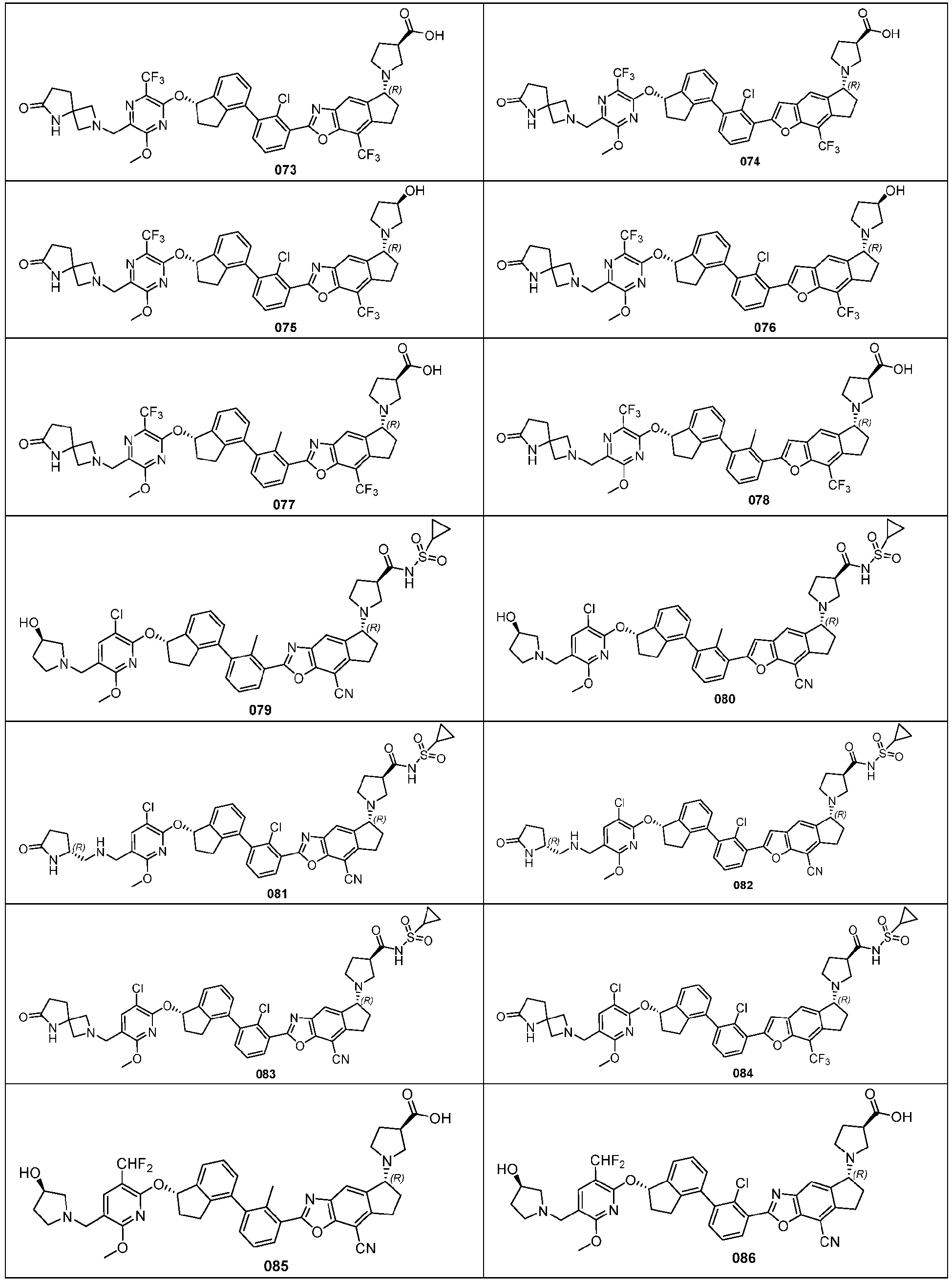

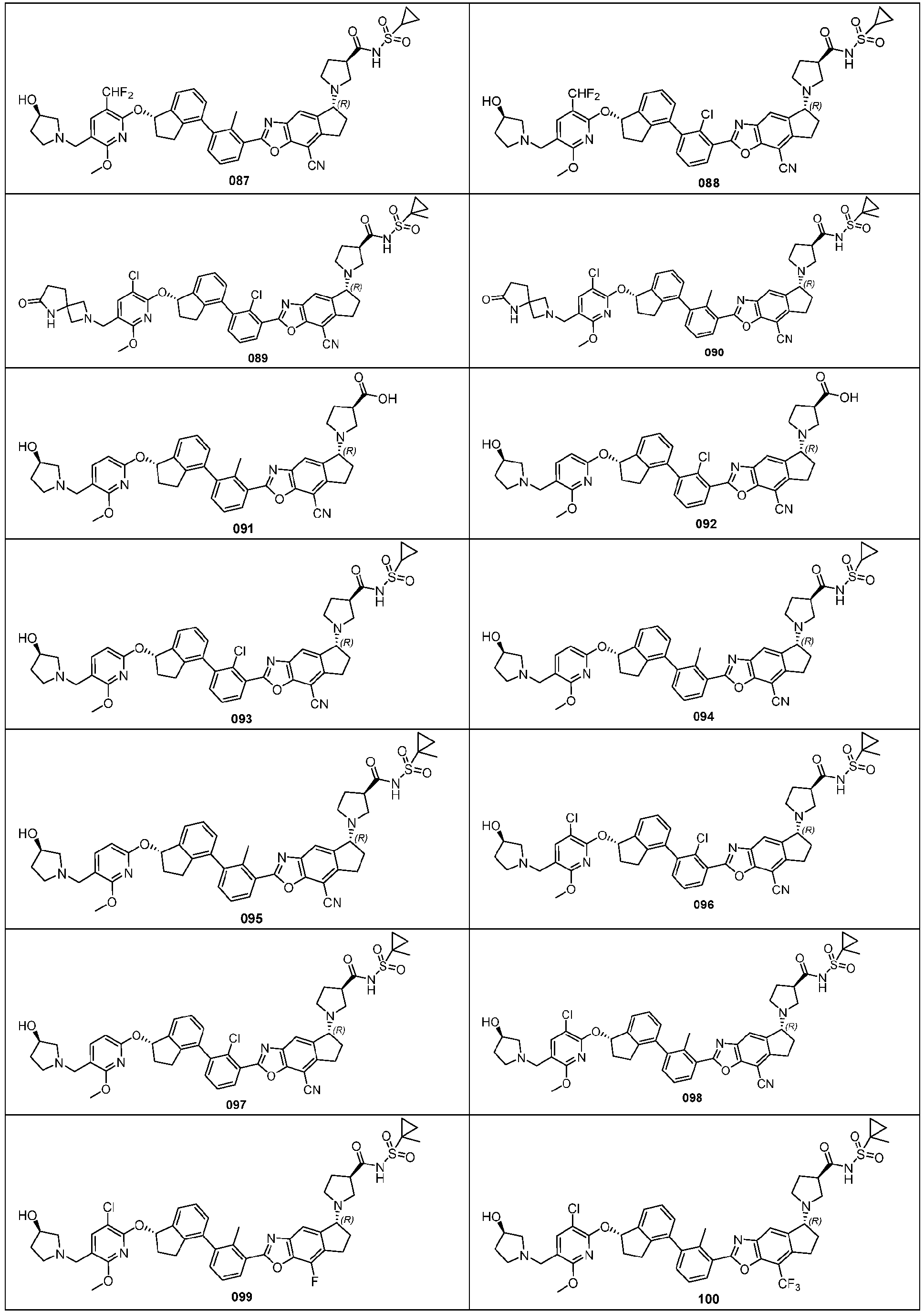

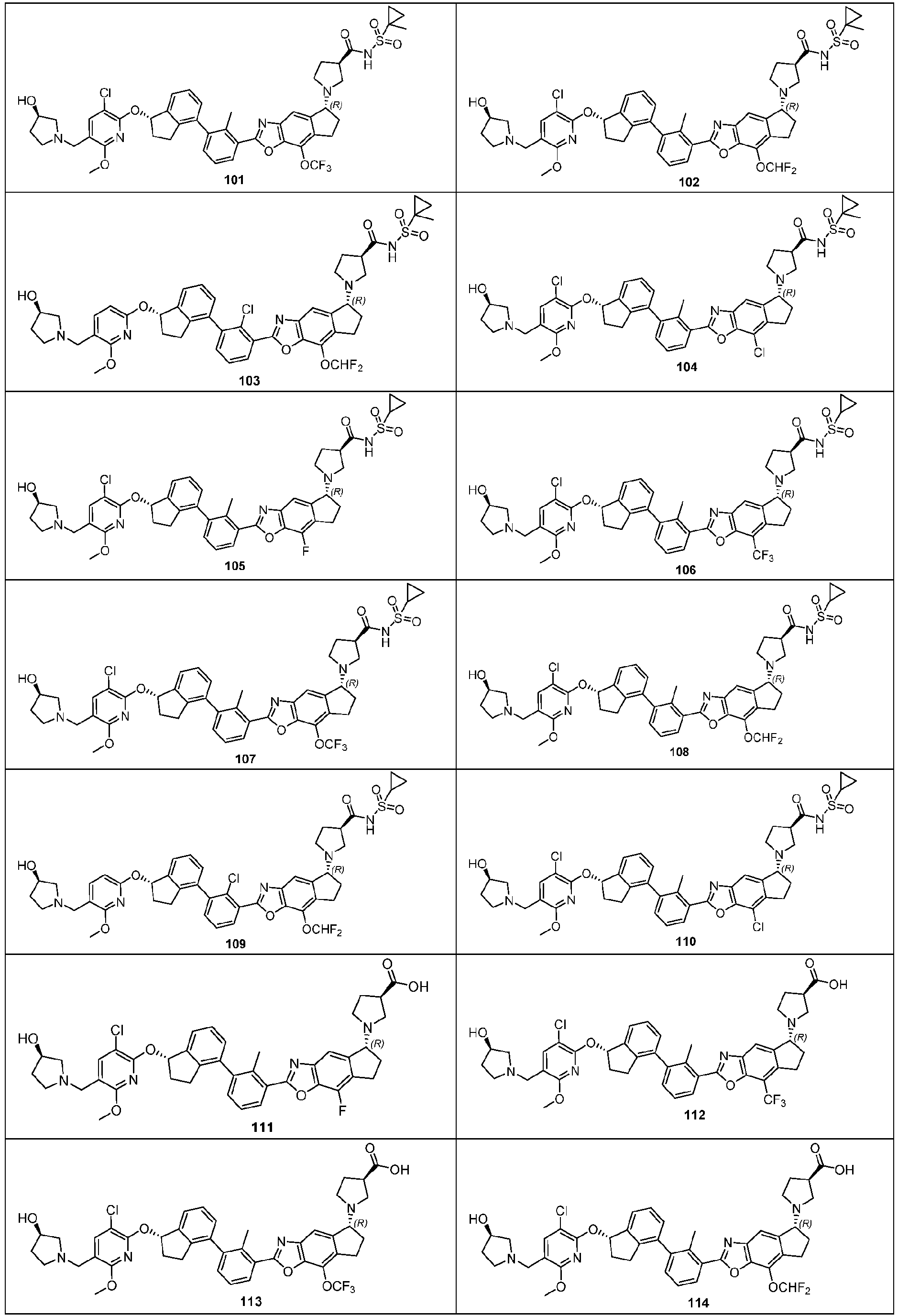

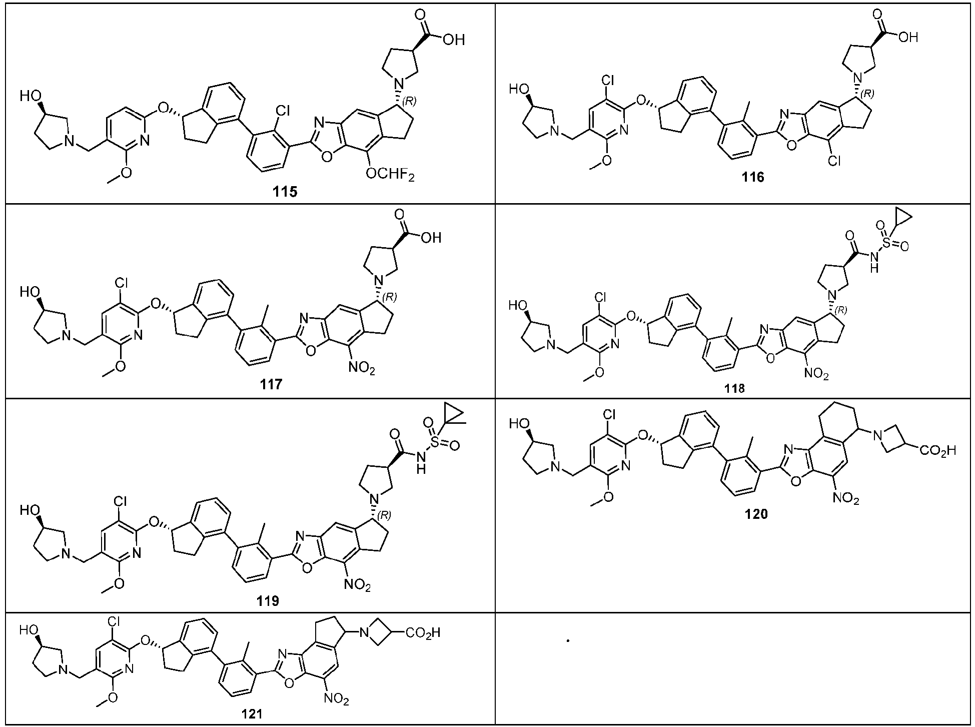

The second aspect of the present invention provides a preparation method of a compound of formula I according to the first aspect of the present invention, comprising steps selected from the steps shown in Synthesis Scheme 1 or 2: 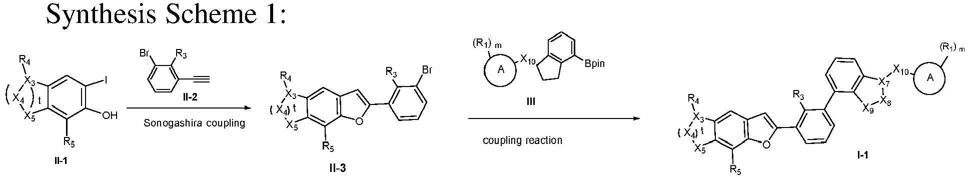
(a) subjecting intermediates II-1 and II-2 as raw materials to Sonogashira coupling reaction catalyzed by palladium catalyst to obtain intermediate II-3;
(b) subjecting II-3 and III-1 as raw materials to a coupling reaction (such as Suzuki, Buchwald, etc.) in the presence of palladium catalyst and ligand to obtain intermediate I-1;
   preferably, the preparation method of intermediate II-1 is as follows: 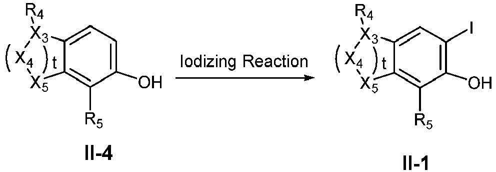 subjecting II-4 as raw material to a halogenation reaction catalyzed by Lewis acid to obtain intermediate II-1;
   preferably, the preparation method of intermediate III-1 is as follows: 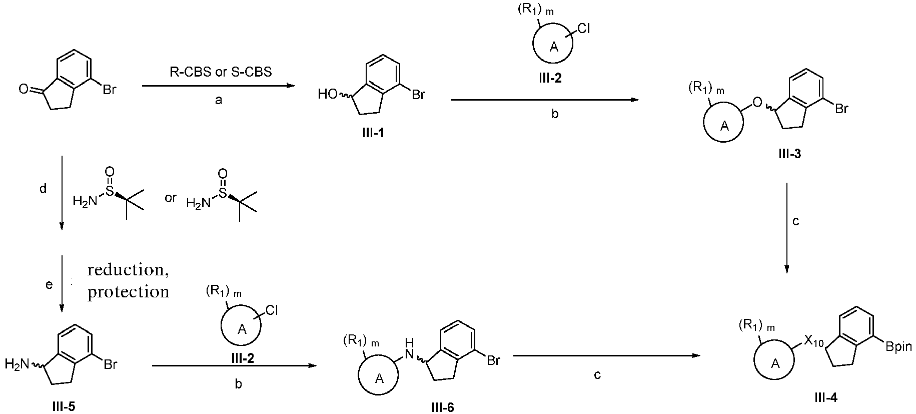
   (a) reacting compound 4-bromoindanone as a raw material under the action of chiral auxiliary (such as R/S-CBS) and reducing agent (such as borane) to form chiral alcohol 1-2;
   (b) subjecting **III-1** (or **III-5)** and **III-2** as raw materials to an affinity substitution reaction under basic condition to obtain intermediate **III-3** (or **III-6);**
   (c) subjecting **III-3** (or **III-6)** and bis(pinacolato)diboron as raw materials to a boronation reaction under the presence of palladium catalyst and ligand to obtain intermediate **III-1;**
   (d), (e) reacting compound 4-bromoindanone and R/S-tert-butylsulfonimide as raw materials under the action of Lwesis acid (such as ethyl tetratitanate) and reducing agent (such as lithium aluminum tetrahydride, sodium borohydride, etc.) to form a protected chiral amino compound, and then the protective group is removed under acidic condition to obtain a chiral amino compound **III-5;**

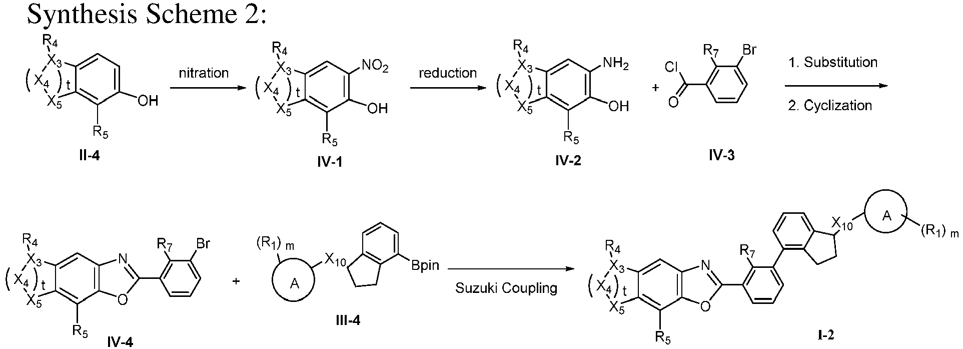

   (a) reacting compound **II-4** as a raw material with a nitrifying agent (such as concentrated sulfuric acid/NaNO₃, concentrated sulfuric acid/fuming nitric acid, etc.) to form intermediate IV-1;
   (b) subjecting **IV-1** as a raw material to a reduction reaction under reducing condition (Pd-C/H₂; zinc powder/ammonium chloride; iron powder/acetic acid etc.) to form intermediate **IV-2**;
   (c) subjecting **IV-2** and **IV**-**3** as raw materials to an affinity substitution reaction under alkaline conditionan to obtain amide intermediate; and then subjecting to a cyclization reaction in the presence of suitable dehydration reagent (such as PPh₃/DDQ) to obtain an intermediate **IV-4**;
   (d) subjecting **IV-4** and IV**-5** as raw materials to a coupling reaction under the conditions of catalyst and ligand to form target product **I-2**;
      X₁-X₁₀, R₁-R₁₀, t, and m are defiend as above.

The third aspect of the present invention provides a pharmaceutical composition, the pharmaceutical composition comprises (1) the compound according to the first aspect of the present invention or a stereoisomer or a tautomer thereof, or a pharmaceutically acceptable salt, a hydrate or a solvate thereof; (2) a pharmaceutically acceptable carrier.

The fourth aspect of the present invention provides a use of the compound according to the first aspect of the present invention or a stereoisomer or a tautomer thereof or a pharmaceutically acceptable salt, a hydrate or a solvate thereof, or a pharmaceutical composition according to the third aspect of the present invention, for the preparation of a pharmaceutical composition for preventing and/or treating diseases related to the activity or expression of PD-1/PD-L1.

The fifth aspect of the present invention provides a PD-1/PD-L1 modulator, which comprises the compound according to the first aspect of the present invention, or a stereoisomer or a tautomer thereof, or a pharmaceutically acceptable salt, hydrate or solvate thereof.

In another preferred embodiment, the pharmaceutical composition is used to treat a disease selected from the group consisting of cancer, infectious disease, and autoimmune disease.

In another preferred embodiment, the cancer is selected from the group consisting of pancreatic cancer, bladder cancer, colorectal cancer, breast cancer, prostate cancer, kidney cancer, hepatocellular carcinoma, lung cancer, ovary cancer, cervical cancer, stomach cancer, esophageal cancer, melanoma, neuroendocrine cancer, central nervous system cancer, brain cancer, bone cancer, soft tissue sarcoma, non-small cell lung cancer, small cell lung cancer or colon cancer, skin cancer, lung cancer, urinary system tumor, blood tumor, glioma, digestive system tumor, reproductive system tumor, lymphoma, nervous system tumor, brain tumor, head and neck cancer.

In another preferred embodiment, the cancer is selected from the group consisting of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disorder (MPD), chronic myeloid leukemia (CML), multiple myeloma (MM), non-hodgkin lymphoma (NHL), mantle cell lymphoma (MCL), follicular lymphoma, waldestrom macroglobulinemia (WM), T-cell lymphoma, B-cell lymphoma, and diffuse large B-cell lymphoma (DLBCL).

In another preferred embodiment, the infectious disease is selected from bacterial infection and viral infection.

In another preferred embodiment, the autoimmune disease is selected from the group consisting of organ-specific autoimmune disease and systemic autoimmune disease.

In another preferred embodiment, the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, acute idiopathic polyneuritis.

In another preferred embodiment, the systemic autoimmune diseases include rheumatoid arthritis, systemic lupus erythematosus, systemic vasculitis, scleroderma, pemphigus, dermatomyositis, mixed connective tissue disease, autoimmune hemolytic anemia.

In another preferred embodiment, the pharmaceutical composition is also used to improve T cell function in a patient with chronic hepatitis B (CHB).

In another preferred embodiment, the inhibitor further comprises at least one therapeutic agent selected from the group consisting of nivolumab, pembrolizumab, atezolizumab and ipilimumab.

The sixth aspect of the present invention provides a method for regulating the interaction of PD-1/PD-L1 *in vitro,* which comprises the steps of: contacting the compound according to the first aspect of the present invention, or a stereoisomer or a tautomer thereof, or a pharmaceutically acceptable salt, a hydrate or a solvate thereof with a PD-L1 protein.

It should be understood that, within the scope of the present invention, the above technical features of the present invention and the technical features specifically described in the following descriptions (such as the examples) can be combined with each other to form a new or preferred technical solution. Due to space limitations, they will not be repeated herein.

### DETAILED DESCRIPTION OF THE INVENTION

After extensive and intensive research, the present inventors discovered a class of PD-1/PD-L1 interaction modulators with excellent regulatory effect. The present invention has been completed on this basis.

### DEFINITIONS

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C₂-C₆ alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyl group having 2-6 carbon atoms, such as ethynyl, propynyl, butynyl, and the like.

As used herein, the term "C₃-C₁₀ cycloalkyl" refers to cycloalkyl groups having 3 to 10 carbon atoms. It may be a monocyclic ring, such as cyclopropyl, cyclobutyl, cyclopentyl, cyclohexyl, and the like. It may also be in bicyclic form, such as bridged or spiro ring form.

As used herein, the term "C₁-C₈ alkylamino" refers to amine groups substituted with C1-C8 alkyl group, which may be mono- or di-substituted; for example, methylamino, ethylamino, propylamino, isopropylamine, butylamine, isobutylamine, tert-butylamine, dimethylamine, diethylamine, dipropylamine, diisopropylamine, dibutylamine, diisobutylamine, di-tert-butylamine, and the like.

As used herein, the term "C₁-C₈ alkoxy" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-10 membered heterocycloalkyl having 1-3 heteroatoms selected from the group consisting of N, S and O" refers to a saturated or partially saturated cyclic group having 3-10 atoms, wherein 1-3 atoms are heteroatoms selected from the group consisting of N, S and O. It may be a monocyclic ring or in a bicyclic form, such as bridged or spiro ring form. Specific examples may be oxetane, azetidine, tetrahydro-2H-pyranyl, piperidinyl, tetrahydrofuranyl, morpholinyl, pyrrolidinyl, and the like.

As used herein, the term "C₆-C₁₀ aryl" refers to aryl groups having 6 to 10 carbon atoms, such as phenyl, naphthyl, and the like.

As used herein, the term "5-10 membered heteroaryl having 1-3 heteroatoms selected from the group consisitng of N, S and O" refers to cyclic aromatic groups having 5-10 atoms, of which 1-3 atoms are selected from the group consisting of N, S and O. It may be a monocyclic ring or in a fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl, and the like.

Unless otherwise specified, the group described in the present invention is "substituted or unsubstituted", the group of the present invention can be substituted by a substituent selected from the group consisting of halogen, nitrile, nitro, hydroxy, amino, C₁-C₆ alkyl-amine, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkyl-sulfonyl, etc.

As used herein, "halogen" or "halogen atom" refers to F, Cl, Br, and I. More preferably, the halogen or halogen atom is selected from F, Cl and Br. "Halogenated" means substitution with an atom selected from F, Cl, Br, and I.

Unless otherwise specified, the structural formula described herein are intended to include all isomeric forms (such as enantiomeric, diastereomeric, and geometric isomers (or conformational isomers)): for example, R, S configuration of asymmetrical centers, (Z), (E) isomers of double bonds, etc. Therefore, the single stereochemical isomers or enantiomers, diastereomers or geometric isomers (or conformers) of the compounds of the invention, or mixtures thereof all fall within the scope of the invention.

As used herein, the term "tautomer" means that structural isomers having different energies can exceed the low energy barrier and thereby transform between each other. For example, proton tautomers (proton shift) include interconversion by proton transfer, such as 1H-carbazole and 2H-carbazole. Valence tautomers include interconversion through some bonding electron recombination.

As used herein, the term "solvate" refers to a complex formed by coordinating a compound of the invention with a solvent molecule in specific proportion.

As used herein, the term "hydrate" refers to a complex formed by coordinating a compound of the invention with water.

### Active ingredient

As used herein, "compounds of the present invention" refers to compounds of formula I, and also includes various crystalline forms, pharmaceutically acceptable salts, hydrates or solvates of the compounds of formula I.

Preferred compounds of the present invention include compounds 1-360 (including various R- and/or S-configuration stereoisomers, and/or E-/Z- cis-trans isomers of each compound).

In another preferred embodiment, the pharmaceutically acceptable salts include salts formed by combining with inorganic acids, organic acids, alkali metal ions, alkaline earth metal ions or organic bases capable of providing physiologically acceptable cations and ammonium salts.

In another preferred embodiment, the inorganic acids are selected from hydrochloric acid, hydrobromic acid, phosphoric acid or sulfuric acid; the organic acids are selected from methanesulfonic acid, p-toluenesulfonic acid, trifluoroacetic acid, medlaric acid, maleic acid, tartaric acid, fumaric acid, citric acid or lactic acid; the alkali metal ions are selected from lithium ion, sodium ion, potassium ion; the alkaline earth metal ions are selected from calcium ion, magnesium ion; and the organic bases capable of providing physiologically acceptable cations are selected from methylamine, dimethylamine, trimethylamine, piperidine, morpholine or tris(2-hydroxyethyl)amine.

All of these salts within the scope of the present invention can be prepared using conventional methods. During the preparation of the compounds of general formula I, solvates and salts thereof, polycrystalline or co-crystal may occur under different crystallization conditions.

The starting materials and intermediates during the preparation of the compound of the present invention are easily obtained, and each step of the reaction can be easily synthesized according to the reported literature or by conventional methods in organic synthesis for those skilled in the art. The compounds of formula I can exist in the form of solvates or nonsolvates, and different solvates may be obtained by crystallization from different solvents.

### Pharmaceutical composition and administration method

Since the compounds of the present invention have excellent inhibitory activity against PD-1/PD-L1 interaction, the compound of the present invention and various crystal forms thereof, pharmaceutically acceptable inorganic or organic salts, hydrates or solvates thereof, and pharmaceutical composition containing the compound according to the present invention as main active ingredient can be used to prevent and/or treat (stabilize, alleviate or cure) a disease associated with PD-1/PD-L1 interaction (eg, cancer, infectious disease, autoimmune disease).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg compounds of the invention per dose, preferably, 10-200mg compounds of the invention per dose. Preferably, the "dose" is a capsule or tablet.

"Pharmaceutically acceptable carrier" means one or more compatible solids or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral administration, parenteral (intravenous, intramuscular or subcutaneous) administration.

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds (such as other anticaner agents).

In the case of co-administration, the pharmaceutical composition can also include one or more (2, 3, 4, or more) other pharmaceutically acceptable compounds. One or more (2, 3, 4, or more) other pharmaceutically acceptable compounds may be used simultaneously, separately or sequentially with the compound of the present invention for the prevention and/or treatment of PD-1/PD-L1 interation related diseases.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000 mg, preferably 20-500mg. Of course, the particular dose should also depend on various factors, such as the route of administration, patient healthy status, which are well within the skills of an experienced physician.

### The main advantages of the present invention include:

(1) The compounds of the present invention have high inhibitory activity on PD-1/PD-L1 interaction, strong binding ability to PD-L1 protein, and the ability to relieve the inhibition of IFNγ by PD-L1.
(2) The compounds of the present invention have better solubility; low toxicity to normal cells, so they can be administered to a subject in a larger dosage range.
(3) Compared with the compounds of the prior art, the compounds of the present invention have better solubility, so they have good druggability. Compared with the existing compounds, the compounds of the present invention show good bioavailability in *in vivo* experiments. In addition, compared with existing compounds, the compounds of the present invention can be easily made into pharmaceutically acceptable salts, thus facilitating further formulation.
*(4) In vivo* pharmacodynamic studies show that the compounds of the present invention can significantly inhibit the growth of subcutaneous tumors in terms of tumor volume and weight, and can significantly increase the number of lymphocytes in the blood and spleen of mice.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The experimental materials and reagents used in the following examples can be commercially available unless otherwise specified.

### General Materials and Test Methods:

The instruments and raw materials involved in the examples are described as follows:
H NMR spectra were obtained by Bruker AV-400 (400MHz) NMR analysis.
Chemical shifts are reported with tetramethylsilane as an internal standard and are expressed in ppm (CDCl₃: δ 7.26 ppm). The recorded data information is as follows: chemical shifts and their splitting and coupling constants (s: singlet; d: doublet; t: triplet; q: quartet; br: broad; m: multiplet).
Mass spectral data were analyzed, among other things, using a LC/MS spectrometer (Finnigan LCQ Advantage), and all reactions were run under dry argon protected anhydrous and oxygen-free conditions. The solid metal organic compounds were stored in an argon-protected dry box.
Tetrahydrofuran and ether were obtained by distillation, in which sodium metal and benzophenone were added. Dichloromethane, pentane and hexane were treated with calcium hydride.

The special raw materials and intermediates involved in the present invention are customized and provided by Tianjin Changsen Pharmaceutical Co., Ltd., etc., and all other chemical reagents are purchased from reagent suppliers such as Shanghai Chemical Reagent Company, Aldrich, and Acros, etc.. If the intermediates or products required for the reaction in the synthesis process are not enough for the next step test, the synthesis is repeated several times to sufficient amount.

Unless otherwise specified, the raw materials and reagents involved in the present invention can be commercially available or can be purchased through customized processing.

The compounds of the present invention may contain one or more asymmetric centers, and thus the series of compounds may be in racemic or single enantiomeric form. The compounds prepared in the present invention are heterocyclic compounds with a purity of more than 95%, and the structural characterization of each final product is determined by MS or/and hydrogen spectral nuclear magnetic resonance (¹H NMR) analysis, respectively. The following examples illustrate the synthesis of various compounds and intermediates of the present invention.

### Example 1 Synthesis of compound 001

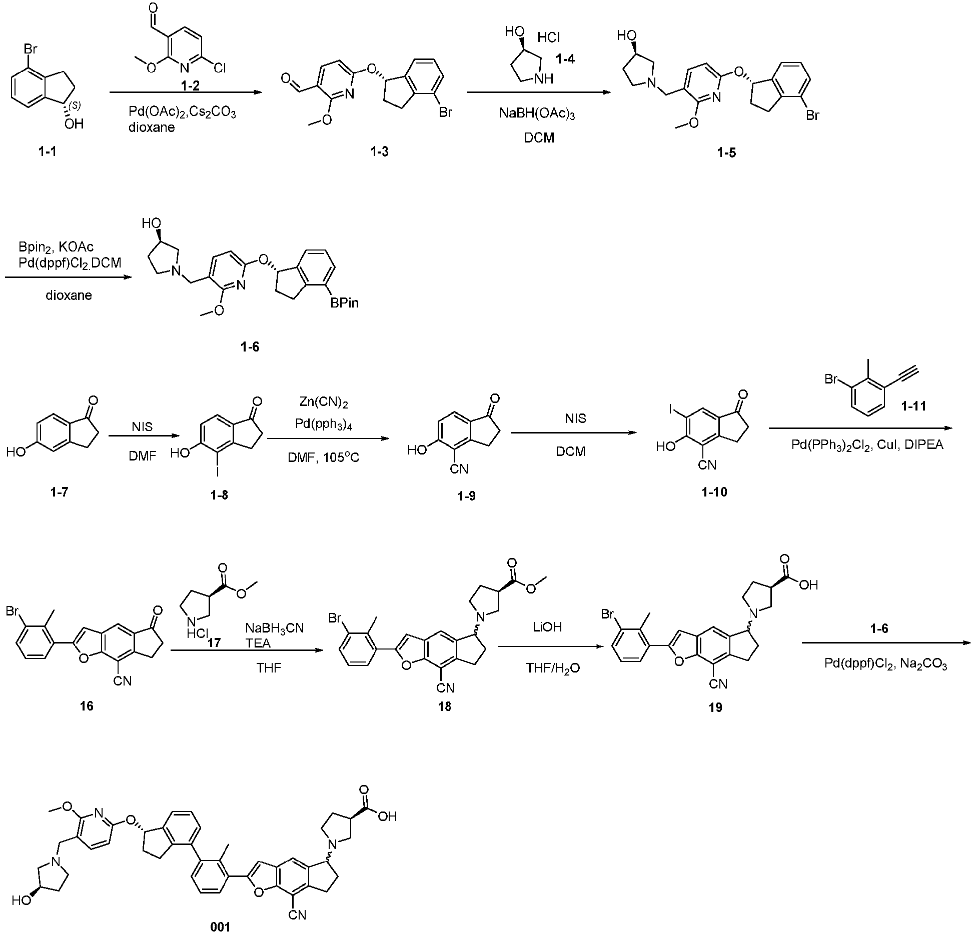

### Step 1-1:

Under the protection of N₂, compound **1-1** (5.0 g, WO2012158550), **1-2** (4.4 g), Pd(OAc)₂ (265 mg) and Cs₂CO₃ (15.4 g) were added to dioxane (100 mL) and the mixture was reacted at 95 °C for 2 hours. TLC spotting plate showed that the reaction was complete. The reaction solution was filtered, and the filtrate was dried by rotary evaporation and subjected to acolumn chromatography (EA:HEP=1:30) to obtain 4.8 g of pale yellow solid. MS-APCI: 348 [M+H]⁺.

### Step 1-2:

Compounds **1-3** (4.8 g), **1-4** (2.5 g, dissolved in 1.5 mL of AcOH) were added to DCM, followed by the addition of 2.8 g of TEA. After stirring at room temperature for 1 h, NaBH(OAc)₃(4.4 g) was added. After 30 minutes, TLC spotting plate showed that the reaction was complete. The reaction solution was washed with saturated NaHCO₃, extracted with DCM and dried by rotary evaporation. The mixture was purified by a column (MeOH:DCM=1:20) to obtain 5.1 g of product as pale yellow oily viscous substance. MS-APCI: 419 [M+H]⁺.

### Step 1-3:

Under the protection of N₂, compound **1-5** (5.1 g), Bpin₂ (3.4 g), Pd(dppf)Cl₂.DCM (498 mg), KOAc (2.4 g) were added to dioxane (100 mL), and the mixture was reacted at 90 °C for 2.5 hour. The reaction was complete as detected by LC-MS. The reaction solution was filtered, washed with water, extracted with DCM, dried, spin-dried, and passed through a column (DCM:MeOH=30:1) to obtain 5.2 g of the product as a gray solid. MS-APCI: 467 [M+H]+.

1H NMR (400 MHz, Chloroform-d) δ 7.85 (d, J = 8.1 Hz, 1H), 7.75 (d, J = 7.3 Hz, 1H), 7.50 (d, J = 7.5 Hz, 1H), 7.21 (t, J = 7.4 Hz, 1H), 6.45 (dd, J = 7.1, 4.5 Hz, 1H), 6.37 (d, J = 8.0 Hz, 1H), 4.58 (s, 1H), 4.22 (s, 2H), 4.01 (s, 3H), 3.38-3.30 (m, 2H), 3.15-3.07 (m, 2H), 2.62-2.53 (m, 2H), 2.45-2.35 (m, 2H), 2.27-2.17 (m, 3H), 1.33 (s, 12H).

### Step 1-4:

**1-7** (5 g) was dissolved in 50 mL of DMF. NIS (7.6 g, dissolved in 20 mL of DMF) was added dropwise at 0 °C, the dropwise addition was completed after 2 h, and then the mixture was reacted at 0 °C for 1 h. The TLC showed that the starting materials was reacted completely. The reaction solution was poured into water, extracted with EA, dried, and passed through a column to obtain 7.0 g of the product as a pale yellow solid. MS-APCI: 275.2 [M+H]⁺.

### Step 1-5:

Under the N₂ protection, **1-8** (3.0g) was dissolved in DMF (30mL) in a 100mL two-necked flask, and Zn(CN)₂(1.3g), Pd(pph₃)₄(2.5g) were added. The mixture was reacted at 105 °C for 1h. The TLC showed that the starting materials was reacted completely. After cooling, the reaction solution was diluted with EA/HEP and filtered. The filtrate was washed with saturated NaCl and extracted with EA. After column purification, 1.5 g of pure product was obtained as a yellow solid. MS-APCI: 174.0 [M+H]⁺.

### Step 1-6:

In a 25mL two-necked flask, **1-9** (400 mg) was suspended in DCM (5 mL), NIS (520 mg) was added at room temperature, and the reaction was performed for 10 minutes. The material disappeared as detected by TLC spotting plate. Posttreatment: water was added to the reaction solution and the mixture was extracted with DCM. The DCM layer was washed with Na₂S₂O₃, dried, and spin-dried to give 600 mg of product as a brown solid. MS-APCI: 300.2 [M+H]⁺.

### Step 1-7:

In a 15mL reaction tube, under the protection of N₂, **1-10** (400 mg), **1-11** (337 mg, WO2018195321), Pd(PPh₃)₂Cl₂ (232 mg), CuI (13 mg), DIPEA (838 mg) were dissolved in DMAc (3 mL), and the mixture was reacted at 85°C for 2h. 15 disappeared as detected by TLC. The reaction solution was washed with water and extracted with EA. EA was dried, spin dried and purified by column to obtain 260 mg of product as a pale yellow solid. MS-APCI: 366.2 [M+H]⁺.

### Step 1-8:

In a 15mL reaction tube, **1-16** (150 mg), **1-17** (135 mg), NaBH₃CN (39 mg), and TEA (83 mg) were added to THF (5 mL), and the mixture was reacted at 70 °C overnight. 16 disappeared as detected by TLC. The reaction solution was spin-dried, and added with water and then EA for extraction. EA was dried, spin dried, and the residue was purified by column to obtain 120 mg of product as a pale yellow oil. MS-APCI: 479.2 [M+H]⁺.

### Step 1-12:

**1-18** (120 mg) was dissolved in THF/H₂O (4 mL, 1:1), LiOH (18 mg) was added, and the reaction was carried out at room temperature for 1 h. 18 disappeared as the detection of TLC. THF was removed from the reaction solution by rotary evaporation, and 2N HCl was added to adjust pH=4. The solid was precipitated, filtered, and spin-dried to obtain 100 mg of the product as a white solid. MS-APCI: 465.2 [M+H]⁺.

### Step 1-9:

Under the protection of N₂, compounds **1-19** (100 mg), **1-6** (110 mg), Pd(dppf)Cl₂ (10 mg), and Na₂CO₃ (43 mg) were added to dioxane/H₂O (2.5 mL, 4:1), and the mixture was reacted at 90 °C 2h. The reaction was complete as detected by TLC. The reaction solution was filtered, washed with water, extracted with EA, dried, spin-dried, and passed through a column (DCM:MeOH=10:1) to obtain 80 mg of the product as a pale yellow solid. MS-APCI: 725.3 [M+H]⁺.

### Example 2 Synthesis of compound 002

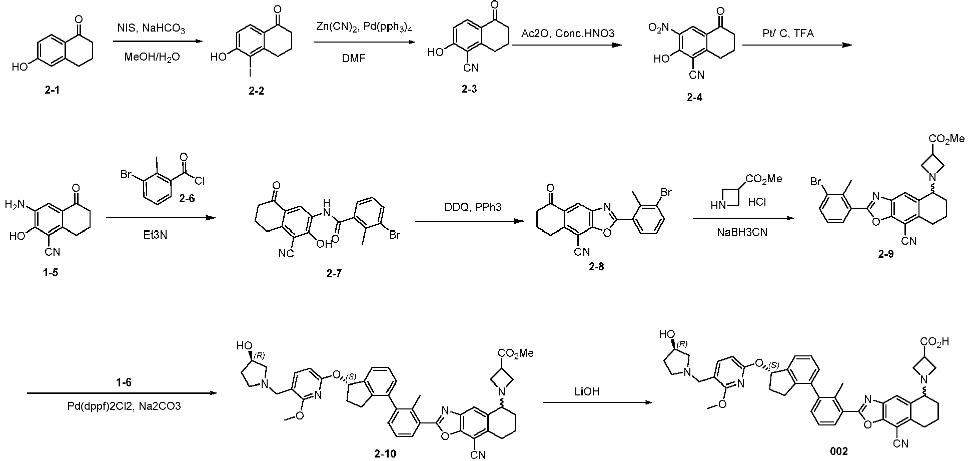

### Step 2-1:

The raw material **2-1** (51 g) was dissolved in 500 ml of methanol, 300 ml of water and sodium bicarbonate (28.9 g) was added, and a solution of NIS in methanol was added at about 0°C. After addtion, the reaction was carried out overnight. Post-processing: the reaction solution was concentrated to dryness, 500 ml of water was added, 3M hydrochloric acid was used to adjust pH to 3. The solid was filtered out, ethyl acetate/ethanol were used to make a slurry, and the mixture was filtered out and dried to obtain 130 g of gray solid.

### Step 2-2:

The raw materials **2-2** (63 g), ZnCN₂ (27 g), and Pd(PPh₃)₄ (12.8 g) were mixed in a 1000 mL three-necked flask, 1000 mL DMF was added, and the atomsphere was replaced by N₂ for 3 times. The mixture was stirred at 90 °C for 3 h. TLC dot plate showed that a small amount of raw material remained, and LCMS showed that the raw material was reacted completely.

### Step 2-3:

Acetic anhydride (75 ml) and concentrated nitric acid (5 ml) were added to a 250 ml single-necked flask. Raw material **2-3** (5.0 g, 28.9 mmol) was added in batches, the mixture was stirred in water bath, and precipitate formed after 20 min. TLC spotting plate showed the reaction was complete. The precipitate was filtered out, drained, slurried, and pulled to dryness to obtain 4 g of white solid.

### Step 2-4:

THF (200 ml), raw material **2-4** (5.0 g), TFA (0.2 mL) and Pt/C were added to a 500 ml single-necked flask, the atomsphere was replaced by H₂ for 3 times. The mixture was stirred at room temperature, reacted for 1 hour. As monitored by TLC spotting plate, the raw material was reacted completely. Pt/C was filtered out, and the filtrate was directly used in the next step.

### Step 2-5:

180 mmol Et₃N was added to the solution of the above filtrate **2-5** (11 g) in THF, to which the solution formed by the dissolution of **2-6** (11.8 g, Organic Letters, 2019, 21, 5971-5976*)* in 15 ml of DCM was added. The mixture was reacted at room temperature for 1 hour to form a lot of turbidity. The solid was filtered off and slurried with ethyl acetate and purified to give 15 g of an off-white solid.

### Step 2-4:

PPh₃ (9.7 g) was dissolved in toluene at room temperature, DDQ (8.4 g) was slowly added, and the resluting mixture was mixed well, at this time the system is suspended. Raw material **2-7** (8 g) was added and the mixture was reacted at 110 °C for 1 hour. The product is mainly by TLC spotting plate. The clear liquid was poured out, concentrated to dryness, and slurried with EA to obtain 3.7g of white solid.

### Step 2-5:

In a 15mL reaction tube, **2-8** (1 g), methyl 3-acridinecarboxylate (0.6 g), NaBH₃CN (0.1 g), TEA (83 mg) were added to THF (5 mL), and the mixture was reacted at 70 °C for 5h. 1-6 disappeared by TLC detection. The reaction solution was spin-dried, and water was added for EA extraction. EA was dried, spin dried, and purified by column to obtain 0.5 g of yellow solid.

### Step 2-6:

Bromine raw materials **2-9** (1.7 g), **1-6** (1 g), Pd(dppf)Cl₂ (0.12 g) and sodium carbonate (0.62 g) were taken and added into a 50 mL reaction flask, the atomsphere was replaced by nitrogen for 3 times. 1,4-Dioxane (8 mL) and water (2 mL) were added and the mixture was reacted at 90 °C; TLC tracking; post-processing: saturated ammonium chloride was added to quench under ice bath. The reaction solution was extracted 2 times with MTBE, washed 5 times with saturated brine. samples were mixed, passed through the column (HEP-DCM=100, 50:1, 10:1, 5:1) to wash down the product and the product was concentrated to obtain 0.6 g of a yellow solid. MS-APCI: 740 [M+H]⁺

### Step 2-7:

**2-10** (53 mg) was added in 5 mL of MeOH, 2 mL of NaOH (2N) was added, and the mixture was reacted at 26 °C for 5 hours. The reaction solution was spin-dried at 40 °C, 50 mL of water was added to the residual solid, and the pH value was adjusted to 7 with 2M HCl. The solid was filtered out, washed with water, washed with THF, dried, and further subjected to preparation purification to obtain 5 mg of yellow solid. MS-APCI: 726 [M+H]⁺.

### Example 3 Synthesis of compound 003

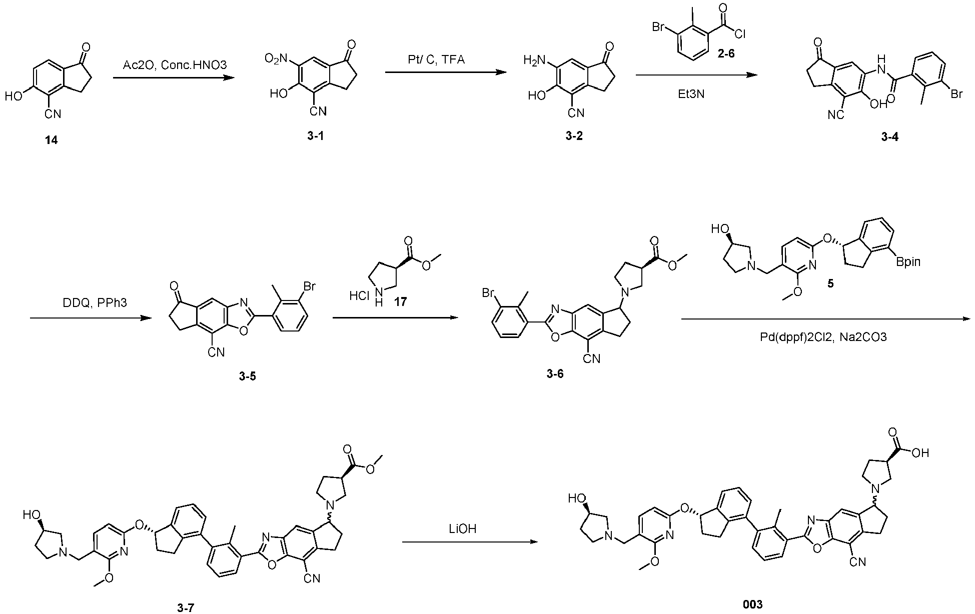

### Step 3-1:

To a 250 ml single-necked flask, acetic anhydride (75 ml), concentrated nitric acid (5 ml) were added and raw material 14 (5.0 g, 28.9 mmol) was added in batches. The mixture was stirred under a water bath and precipitate was formed after 20 min. As monitored by TLC spotting plate, the reaction was completed. The precipitate was filtered out, drained, beaten to slurry, and pulled to dryness to obtain 4.5 g of white solid with a yield of 71%.

### Step 3-2:

To a 500 ml single-necked flask, THF (200 ml), raw material **3-1** (5.0 g, 23 mmol) and TFA (0.2 mL), Pt/C were added, the atomsphere was replaced by H₂ for 3 times. The mixture was stirred at room temperature. After the reaction was performed for 1 hour, the raw materials was reacted to complete by TLC spot plate monitoring. Pt/C was filtered out, and the filtrate was directly used in the next step.

### Step 3-3:

To the solution of the above filtrate **3-2** (10.0 g, 45.8 mmol) in THF was added 180 mmol Et₃N, to which the solution formed by the dissolution of **2-6** (10.74 g, 46 mmol, Organic Letters, 2019, 21, 5971-5976) in 15 ml of DCM was slowly added. The mixture was reacted at room temperature for 1 hour, forming a lot of turbidity. The solid was filtered off and slurried with ethyl acetate and purified to give 18 g of off-white solid.

### Step 3-4:

PPh₃ (9.7 g, 37 mmol) was dissolved in toluene at room temperature, DDQ (8.4 g, 37 mmol) was slowly added, and the resluting mixture was mixed well. At this time the system is suspended. Raw material **3-4** (7.1 g, 18.5 mmol) was added, and the mixture was reacted at 110 °C for 1 hour. The product is mainly by TLC spotting plate. The clear liquid was poured out, concentrated to dryness, and slurried with EA to obtain 3.7g of white solid.

### Step 3-5:

0.8 g of white solid was obtained according to the synthetic method of step 1-11 using compound **3-5** (1 g, 2.72 mmol) and **17** (902 mg, 5.45 mmol) as raw materials. MS (APCI): 480 [M+H]⁺

### Step 3-6:

0.21 g of white solid was obtained according to the synthesis method of steps 1-12 using compound **3-6** (200 mg, 0.416 mmol) and **5** (233 mg, 0.5 mmol) as raw materials. MS (APCI): 740 [M+H]+

### Step 3-7:

Compound **3-7** (210 mg, 0.284 mmol) and LiOH (20 mg, 0.85 mmol) were successively added to a single-necked flask containing THF/MeOH/H₂O (2:2:1, 5 mL), and the mixture was stirred at room temperature for 2 hours. The reaction was detected by TLC. After the reaction was completed, 50 mg of white solid was obtained by using a reversed-phase column. MS (APCI): 726 [M+H]⁺

### Example 4 Synthesis of compound 004

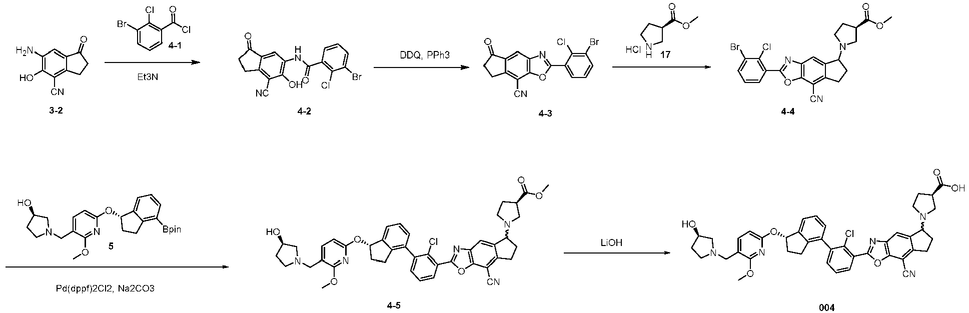

### Step 4-1:

3 g of off-white solid was prepared according to the synthesis method of step 3-3 using compound **3-2** (2 g, 0.106 mmol) and **4-1** (2.83 g, 0.011 mmol, WO2017059135) as raw materials. MS (APCI): 405 [M+H]⁺

### Step 4-2:

1.1 g of off-white solid was prepared according to the synthesis method of step 3-4 using compound **4-2** (2 g) as a raw material. MS (APCI): 387 [M+H]⁺

### Step 4-3:

0.9 g of off-white solid was prepared according to the synthesis method of step 3-5 using compound **4-3** (1 g, 2.58 mmol) and compound **17** (0.85 g, 5.16 mmol) as raw materials. MS (APCI): 500 [M+H]⁺

### Step 4-4:

0.18 g of off-white solid was prepared according to the synthesis method of step 3-6 using compound **4-4** (0.2 g, 0.4 mmol) and compound **5** (0.22 g, 0.48 mmol) as raw materials. MS (APCI): 760 [M+H]⁺

### Step 4-5:

42 mg of white solid was prepared according to the synthesis method of step 3-7 using compound **4-5** (0.2 g, 0.4 mmol) as a raw material. MS (APCI): 746 [M+H]+

### Example 5 Synthesis of compound 005

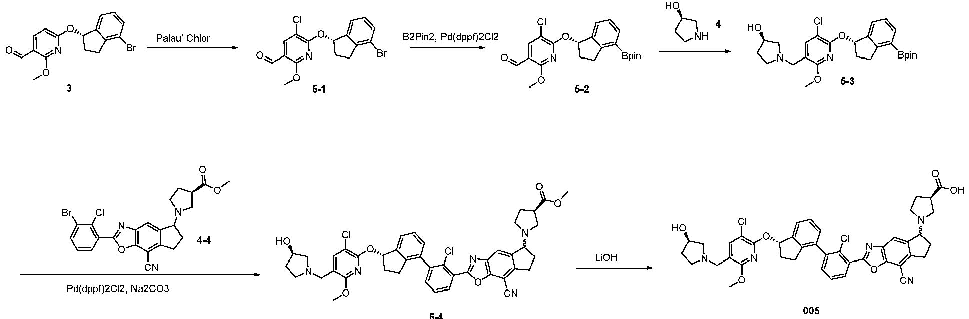

### Step 5-1:

Compound **3** (1.5 g, 4.34 mmol), Palau' Chlor (1 g, 4.77 mmol) and TFA (0.35 mL, 4.77 mmol) were successively added to the single-necked flask containing CH₃CN/DCM (2:1, 30 mL). After stirred at room temperature overnight, the reaction was detected by LC-MS. After the reaction was completed, the reaction solution was filtered and rinsed with DCM twice, then the filtrate was washed with saturated aqueous sodium bicarbonate solution and brine, dried over anhydrous sodium sulfate, and subjected to column chromatography to obtain 1.46 g of white solid. MS-APCI: 188 [M-195+H]⁺ (fragmentation peak)

### Step 5-2:

Compound **5-1** (1.4 g, 3.66 mmol), B2pin2 (1.02 g, 4 mmol), KOAc (718 mg, 7.32 mmol), Pd(dppf)₂Cl₂ (257 mg, 0.366 mmol) were successively added to a single-neck flask containing dioxane (30 mL). Under the protection of nitrogen, the mixture was heated and stirred at 95 °C in an oil bath for 3 hours, and the reaction was detected by LC-MS. After the reaction was completed, the reaction solution was filtered, spin-dried. The samples were mixed, and subjected to column chromatography to obtain 800 mg of oil. MS-APCI: 188 [M-243+H] ⁺ (fragment peak)

### Step 5-3:

Compound **5-2** (800 mg, 1.86 mmol), R-3-hydroxytetrahydropyrrole hydrochloride (460 mg, 3.72 mmol), DIPEA (0.3 mL, 1.86 mmol) and NaBH(OAc)3 (1.18 g, 5.59 mmol) were successively added to a single-necked flask containing DCM (30 mL), the mixture was stirred at room temperature for 2 hours, and the reaction was detected by TLC. After the reaction was completed, the reaction solution was washed with saturated aqueous sodium bicarbonate solution and brine, dried over anhydrous sodium sulfate, and subjected to column chromatography to obtain 670 mg of white solid. MS-APCI: 501 [M+H]⁺

### Step 5-4:

160 mg of white solid was obtained according to the synthesis method of steps 1-12 using compound **5-3** (200 mg, 0.4 mmol) and **4-4** (160 mg, 0.32 mmol) as raw materials. MS-APCI: 794 [M+H]⁺

### Step 5-5:

21 mg of white solid was prepared according to the synthetic method of step 3-7 using compound **5-4** (160 mg) as raw material. MS (APCI): 780 [M+H]+

### Example 6 Synthesis of compound 006

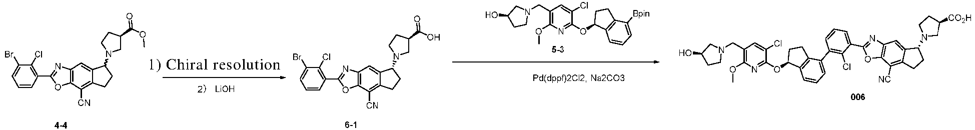

### Step 6-1:

Raw material **4-4** (10 g) was subjected to chiral column resolution to give S-(4-4) (4.2 g). Then, 3 g of white solid was obtained according to the synthesis method of step 3-7 using S-(4-4) as a raw material. MS (APCI): 486 [M+H]⁺

### Step 6-2:

38 mg of white solid was prepared according to the synthesis method of steps 1-12 using raw material **6-1** (200 mg, 0.41 mmol) and 5-3 (247 mg, 0.49 mmol) as raw materials. MS (APCI): 780 [M+H]⁺

### Example 7 Synthesis of compound 007

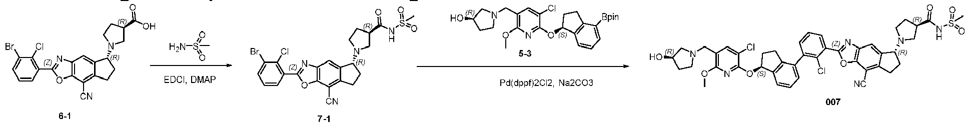

### Step 7-1:

**6-1** (200.0mg 1.0eq), cyclopropylsulfonamide (200.0mg, 4.0eq), DMAP (202.0mg, 4.0eq), EDCI (314.0mg, 4.0eq) were added to the reaction flask and dissolved in 6 mL of DCM, the mixture was stirred at RT for 4h. As monitored by TLC, the starting material was reacted completely. The reaction solution was quenched with 1M HCl, washed with saturated brine, dried, concentrated, and subjected to column chromatography to obtain 180 mg of pale yellow solid. MS (APCI): 563 [M+H]⁺

### Step 7-2:

26 mg of white solid was prepared according to the synthesis method of steps 1-12 using raw materials **7-1** (200 mg, 0.355 mmol) and **5-3** (213 mg, 0.43 mmol) as raw materials. MS (APCI): 857 [M+H]⁺

### Example 8 Synthesis of compound 008

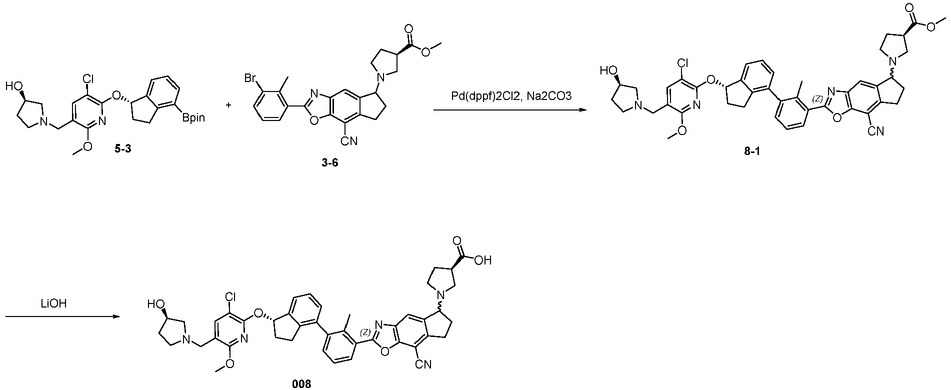

### Step 8-1:

120 mg of white solid was obtained by column chromatography according to the synthesis method of steps 1-12 using raw materials **3-6** (200 mg, 0.41 mmol) and 5-3 (247 mg, 0.49 mmol) as raw materials.

### Step 3-7:

Compound **8-1** (210 mg) and LiOH (18 mg) were sequentially added to a single-necked flask containing THF/MeOH/H₂O (2:2:1, 5 mL), the mixture was stirred at room temperature for 2 hours, and the reaction was detected by TLC. After the reaction was completed, 50 mg of white solid was obtained by using a reversed-phase preparative column. MS (APCI): 760 [M+H]⁺

The compounds in the following table were synthesized by using the synthesis method of compounds 001-008.

| Number | MS [M+H]⁺ | Number | MS [M+H]⁺ | Number | MS [M+H]⁺ |
|---|---|---|---|---|---|
| **009** | 780 | **046** | 822 | **083** | 922 |
| **010** | 760 | **047** | 850 | **084** | 921 |
| **011** | 807 | **048** | 849 | **085** | 776 |
| **012** | 806 | **049** | 822 | **086** | 796 |
| **013** | 779 | **050** | 821 | **087** | 879 |
| **014** | 778 | **051** | 830 | **088** | 899 |
| **015** | 787 | **052** | 829 | **089** | 936 |
| **016** | 786 | **053** | 802 | **090** | 916 |
| **017** | 759 | **054** | 758 | **091** | 726 |
| **018** | 758 | **055** | 862 | **092** | 746 |
| **019** | 819 | **056** | 861 | **093** | 849 |
| **020** | 818 | **057** | 834 | **094** | 829 |
| **021** | 791 | **058** | 833 | **095** | 843 |
| **022** | 790 | **059** | 842 | **096** | 897 |
| **023** | 799 | **060** | 841 | **097** | 863 |
| **024** | 798 | **061** | 814 | **098** | 877 |
| **025** | 771 | **062** | 813 | **099** | 870 |
| **026** | 770 | **063** | 858 | **100** | 920 |
| **027** | 815 | **064** | 838 | **101** | 936 |
| **028** | 814 | **065** | 885 | **102** | 918 |
| **029** | 842 | **066** | 884 | **103** | 904 |
| **030** | 841 | **067** | 857 | **104** | 886 |
| **031** | 814 | **068** | 856 | **105** | 856 |
| **032** | 813 | **069** | 865 | **106** | 906 |
| **033** | 822 | **070** | 864 | **107** | 922 |
| **034** | 821 | **071** | 837 | **108** | 904 |
| **035** | 794 | **072** | 836 | **109** | 890 |
| **036** | 793 | **073** | 897 | **110** | 872 |
| **037** | 854 | **074** | 896 | **111** | 753 |
| **038** | 853 | **075** | 869 | **112** | 803 |
| **039** | 826 | **076** | 868 | **113** | 819 |
| **040** | 825 | **077** | 877 | **114** | 801 |
| **041** | 834 | **078** | 876 | **115** | 787 |
| **042** | 833 | **079** | 863 | **116** | 769 |
| **043** | 806 | **080** | 862 | **117** | 780 |
| **044** | 805 | **081** | 910 | **118** | 883 |
| **045** | 823 | **082** | 909 | **119** | 897 |

### Example 120 Synthesis of Compound 120

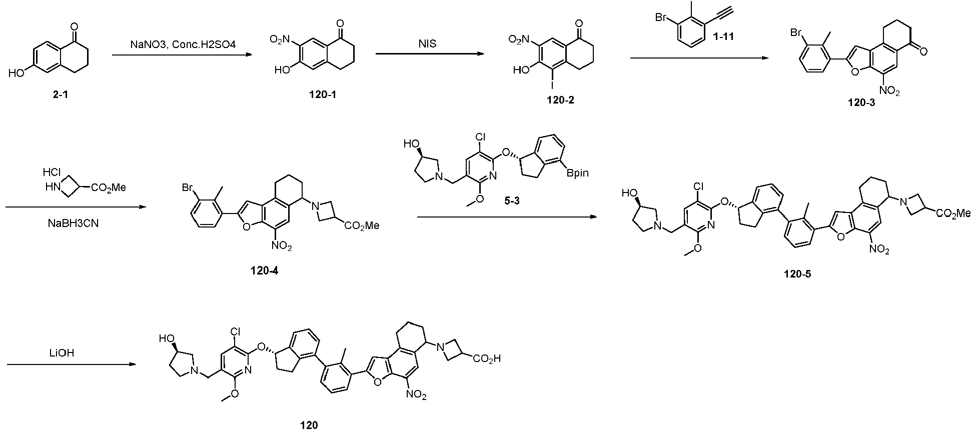

### Step 120-1:

**2-1** (1.3 g) was dissolved in 200 ML of DCM-3M H₂SO₄ (1:1), 0.71 g of sodium nitrate was added to the reaction system under an ice bath at 5 °C. Under nitrogen protection, the mixture was reacted at room temperature overnight. TLC spotting showed 1-1 disappeared, and LCMS analysis showed that the product was formed. The mixture was purified by column chromatography to obtain 300 mg of product. MS-APCI: 208 [M+H]⁺.

### Step 120-2:

Compound **120-1** (4 g) was dissolved in 200 mL of DCM (in a 500 mL one-neck flask), 6.3 g of NIS was added, and the reaction system was protected by argon. The mixture was reacted overnight at room temperature. TLC spotting showed the reaction was complete. 100 mL of 2M HCl was added into the reaction solution, and the resulting solution was separated and dried, spin-dried and purified by column to yield 5 g of product as a pale yellow solid. MS-APCI: 334 [M+H]+.

### Step 120-3:

According to the synthetic method of steps 1-7, the product 200 mg was obtained by using **120-2** (390 mg) and **1-11** (337 mg) as raw materials. MS-APCI: 400 [M+H]⁺

### Step 120-4:

According to the synthetic method of step 2-5, 100 mg of the product was obtained by using **2-8** (200 mg) and methyl 3-acridinecarboxylate (86 mg) as raw materials. MS-APCI: 499 [M+H]⁺

### Step 120-5:

Raw materials **120-4** (100 mg) and **5-3** (110 mg) were used as raw materials, and according to the synthesis method of steps 1-12, 102 mg of white solid was obtained by column chromatography. MS (APCI): 793 [M+H]+

### Step 120-6:

Compound **120-5** (102 mg) and LiOH (20 mg) were sequentially added to a single-necked flask containing THF/MeOH/H₂O (2:2:1, 5 mL), the mixture was stirred at room temperature for 2 hours, and the reaction was detected by TLC. After the reaction was completed, 26 mg of white solid was obtained by using a reversed-phase preparative column. MS (APCI): 779 [M+H]⁺

### Example 121 Synthesis of compound 121

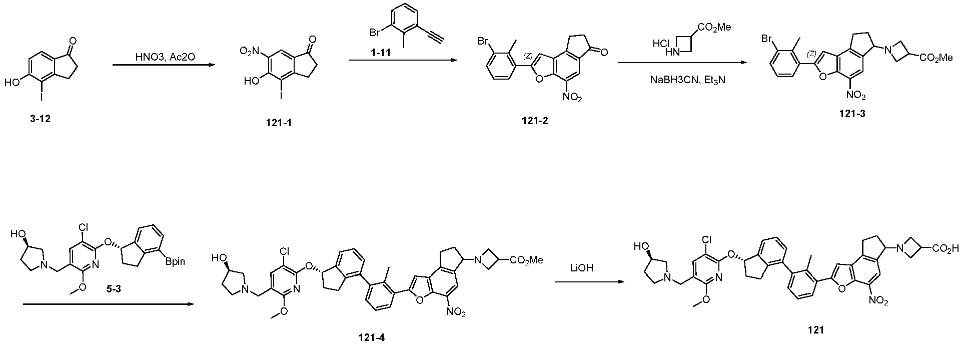

### Step 121-1:

According to the synthesis method of step 3-1, 0.8 g of white solid was prepared by using **3-12** (2 g) as a raw material. MS (APCI): 320 [M+H]⁺

### Step 121-2:

According to the synthetic method of steps 1-7, 615 mg of the product was obtained by using **121-1** (800 mg) and **1-11** (756 mg) as starting materials. MS-APCI: 386 [M+H]⁺

### Step 121-3:

According to the synthetic method of step 2-5, 152 mg of the product was obtained by using **121-2** (200 mg) and methyl 3-acridinecarboxylate (86 mg) as starting materials. MS-APCI: 485 [M+H]⁺

### Step 121-4:

Raw materials **121-3** (100 mg) and **5-3** (108 mg) were used as raw materials. According to the synthesis method of step 1-12, 96 mg of white solid was obtained by column chromatography. MS (APCI): 779 [M+H]⁺

### Step 121-5:

Compound **121-4** (96 mg) and LiOH (15 mg) were sequentially added to a single-necked flask containing THF/MeOH/H₂O (2:2:1, 5 mL), the mixture was stirred at room temperature for 2 hours, and the reaction was detected by TLC. After the reaction was completed, 26 mg of white solid was obtained by using a reversed-phase preparative column. MS (APCI): 765 [M+H]⁺

### Biological Test

### Example A: PD-1/PD-L1 Homogeneous Time-Resolved Fluorescence (HTRF) Binding Assay

Assays were performed in standard black 384-well polystyrene plates and a final volume was 20 µL. The inhibitor was first serially diluted with DMSO and added to the wells of the plate, followed by the addition of other reaction components. The final concentration of DMSO in the assay was 1%. Assays were performed at 25°C in PBS buffer (pH 7.4) containing 0.05% Tween-20 and 0.1% BSA. Recombinant human PD-L1 protein (19-238) with a His-tagged at the C-terminus was purchased from AcroBiosystems (PD1-H5229). Recombinant human PD-1 protein (25-167) with an Fc tag at the C-terminus was also purchased from AcroBiosystems (PD1-H5257). The PD-L1 and PD-1 proteins were diluted in assay buffer and then 0.1 µl of the solution was extracted and added to the wells of the plate. Plates were centrifuged and proteins and inhibitors were preincubated for 40 minutes. After incubation, 0.1 µl of HTRF detection buffer containing europium blocking labeled anti-human IgG (PerkinElmer-AD0212), Fc-specific and anti-His SureLight^{®}-Allophycocyanin (APC, PerkinElmer-AD0059H) conjugated antibodies was added. After centrifugation, the plates were incubated at 25°C for 60 minutes. Data was read in a PHERAstar FS plate reader (665nm/620nm ratio). Final concentrations in the assay were ~3 nM PD1, 10 nM PD-L1, 1 nM europium anti-human IgG, and 20 nM anti-His-allophycocyanin. Activity data were fitted using GraphPad Prism 5_0 software to derive IC50 values for inhibitors.

The IC50 values of the compounds exemplified in the examples are expressed in the following manner: IC50: +: ≤ 10 nM; ++: 10 nM~100 nM; +++: > 100 nM.

The data of the example compounds obtained using the PD-1/PD-L1 homogeneous time-resolved fluorescence (HTRF) binding assay described in Example A showed that the IC50 values of the tested compounds of the present invention are all less than 10 nm, and the IC50 values of some compounds (about 25 %) are even less than 1 nM. The activity test data of some preferred compounds were provided in Table 1.

**Table 1.**

| Compound | PD-1/PD-L1 HTRF IC50(nM) | Compound | PD-1/PD-L1 HTRF IC50(nM) |
|---|---|---|---|
| 001 | + | 087 | + |
| 003 | + | 088 | + |
| 004 | + | 089 | + |
| 005 | + | 090 | + |
| 006 | + | 091 | + |
| 007 | + | 092 | + |
| 079 | + | 093 | + |
| 080 | + | 094 | + |
| 081 | + | 095 | + |
| 082 | + | 096 | + |
| 083 | + | 097 | + |
| 084 | + | 098 | + |
| 085 | + | | |
| 086 | + | | |

All documents mentioned herein are incorporated by reference in the present invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A compound represented by the following formula I, or an optical isomer, a hydrate, a solvate thereof, or a pharmaceutically acceptable salt thereof: 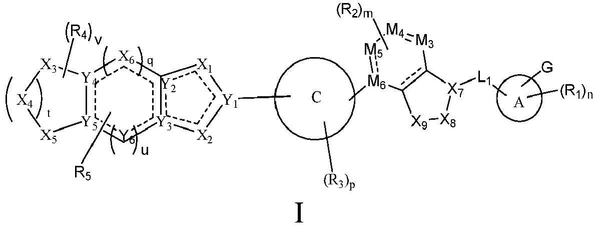 wherein, n, q, m, t, p, v, u are each independently selected from the group consisting of 0, 1, 2, 3, 4, and 5;
X₁ , X₂, X₃, X₄, X₅ and X₆ are each independently selected from the group consisting of N, O, S, SO, SO₂, C(R)₂, CHR, and NR;
Y₁, Y₂, Y₃, Y₄, Y₅ and Y₆ are each independently selected from the group consisting of N, CH, and C;
X₇ is selected from the group consisting of N and CR;
X₈ and X₉ are each independently selected from the group consisting of O, S, SO, SO₂, C(R)₂, NR; wherein, R is selected from the group consisting of H, C₁-C₆ alkyl, chlorine, bromine, fluorine, iodine, cyano, hydroxy, nitro, NRf, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, -C(=O)-substituted or unsubstituted C₂-C₆ alkynyl; wherein, a hydrogen (if present) on a carbon atom of X₃, X₄, X₅, X₇, X₈, X₉ can be each independently substituted by deuterium;
M₃, M₄, and M₅ are each independently selected from the group consisting of: chemical bond, CR, N, NR, O, S, SO, and SO₂;
M₆ is each independently selected from the group consisting of CR, N, and C; and 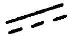 represents single bond or double bond; 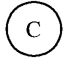 is each independently selected from the group consisting of substituted or unsubstituted C5-C12 arylene, substituted or unsubstituted 5-12 membered (preferably 5-7 membered) heteroarylene having 1-3 heteroatoms, substituted or unsubstituted 5-12 membered heterocyclylene, and substituted or unsubstituted C5-C12 cycloalkylene; 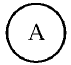
is selected from the group consisting of substituted or unsubstituted 5-12 membered heteroaryl, substituted or unsubstituted C6-C10 aryl, substituted or unsubstituted 3-12 (preferably 5-12) membered heterocyclyl, and substituted or unsubstituted C3-C12 (preferably C5-C12) cycloalkyl, wherein the heterocyclyl has 1-3 heteroatoms;
L₁ is selected from the group consisting of chemical bond, substituted or unsubstituted C1-C4 alkylene, substituted or unsubstituted C2-C4 alkenylene, substituted or unsubstituted C2-C4 alkynylene, -S-, -O-, substituted or unsubstituted -NH-, -S(O)-, -S(O)₂-, substituted or unsubstituted -NHC(O)NH-, 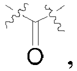 substituted or unsubstituted 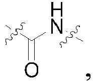 substituted or unsubstituted 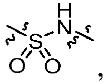 and substituted or unsubstituted 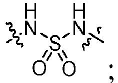
G is absent, H, substituted or unsubstituted C1-C6 alkyl, or 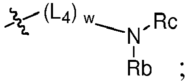 wherein, w is 0, 1, 2, 3, 4, 5, or 6;
each L₄ is independently selected from the group consisting of substituted or unsubstituted C1-C4 alkylene, -S-, -O-, NR_{f}, -S(O)-, -S(O)₂-; preferably substituted or unsubstituted C1-C4 alkylene; wherein, a hydrogen on a carbon atom of the substituted or unsubstituted C1-C4 alkylene may be each independently substituted by deuterium, provided that a structure formed by each L₄ is chemically stable;
R_{b} and R_{c} are each independently selected from the group consisting of H and substituted or unsubstituted C₁-C₈ alkyl; or Rb and Rc together with the adjacent N atom form substituted or unsubstituted 5-10 3-10 membered heterocyclyl having 1-3 heteroatoms selected from N, S and O;
R₁, R₂, R₃, R₄, R₅, G and R are each independently selected from the group consisting of H, -CN, trifluoromethyl, -CHF₂, -OCF₃, -OCHF₂, sulfonamido, nitro, hydroxyl, halogen, -S-R₈, -S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NR_{f}, -CN, hydroxyl, NRdRe (eg amino), substituted or unsubstituted C1-C6 amino, substituted or unsubstituted -(C1-C6 alkylene)-NH-(C1-C6 alkylene), carboxy, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, wherein a hydrogen on a carbon atom of substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e. =O), =NR_{f}, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms can be each independently substituted by deuterium; substituted or unsubstituted 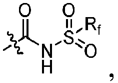 substituted or unsubstituted 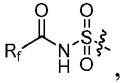 substituted or unsubstituted 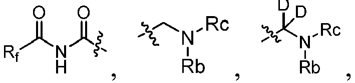 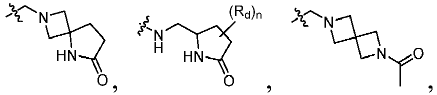 wherein, R_{b}, R_{c} and R_{d} are each independently selected from the group consisting of H and substituted or unsubstituted C₁-C₈ alkyl; or Rb and Rc together with adjacent N atom form substituted or unsubstituted 3-10 membered heterocyclyl having 1-3 heteroatoms selected from N, S and O, or Rb and Rc together with adjacent N atom form substituted or unsubstituted 4-10 membered lactam; wherein, a hydrogen on a carbon atom of Rb, Rc and Rd can be each independently substituted by deuterium; or -(L₁ₐ)ᵣ-(L₂ₐ)ₛ-(L₃ₐ)ₛ-; -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉;
R₈ and R₉ are each independently selected from the group consisting of H, hydroxy, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted-(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 5-12-membered heterocyclyl with 1-4 heteroatoms, substituted or unsubstituted 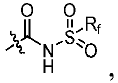 substituted or unsubstituted 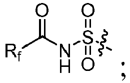 substituted or unsubstituted 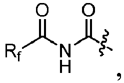 and -(L₁ₐ)ᵣ-(L₂ₐ)ₛ-(L₃ₐ)ₛ-;
each L₁ₐ is independently selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, and -S(O)₂-;
L₂ₐ is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, and substituted or unsubstituted 5-10 membered heterocyclylene with 1-3 heteroatoms;
L₃ₐ is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g}, -OR_{g}, -N(R_{g})₂, -CO₂R_{g}, -CON(R_{g})₂, -CONHCOR_{g}, NR_{g}-CO-N(R_{g})₂, and -NRg-SO₂-N(Rg)₂;
r is 1, 2, 3, 4, 5, or 6;
s is 0, 1, or 2;
R_{d}, Rₑ and R_{g} are each independently selected from the group consisting of H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, and substituted or unsubstituted C₆-C₁₀ aryl; or Rd and Re together form substituted or unsubstituted 3-10 (preferably 5-10) membered cycloalkyl, or 3-10 (preferably 5-10) membered heterocyclyl having 1-3 heteroatoms selected from N, S and O;
R_{f} is selected from the group consisting of H, substituted or unsubstituted C₁-C₆ alkyl, substituted or unsubstituted C₆-C₁₀ aryl, substituted or unsubstituted C₆-C₁₀ heteroaryl, cyano, -C(=O)-NRdRe, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, and -C(=O)-substituted or unsubstituted C₂-C₆ alkynyl;
unless otherwise specified, the "substituted" refers to substitution with one or more (eg 2, 3, 4, etc.) substituents selected from the group consisting of halogen (including -F, Cl, Br), -CH₂Cl, -CHCl₂, -CCl₃, -CH₂F, -CHF₂, -CF₃, oxo =O, - CN, hydroxyl, amino, C1-C6 alkylamino, carboxyl, -NHAc, 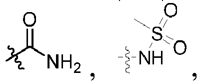 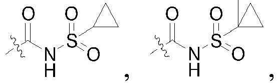 an unsubstituted or substituted group selected from the group consisting of C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, 5-10 membered heterocyclyl with 1-3 heteroatoms selected from N, S and O, which is substituted by one or more substituents selected from the following group; the substituent is selected from the group consisting of halogen, hydroxyl, carboxyl, cyano, C1-C6 alkoxy, and C1-C6 alkylamino;
in the above formulas, any one of the heteroatom is selected from the group consisting of B, P, N, S and O.

2. The compound according to claim 1, or the isomer, optical isomer, hydrate, solvate thereof, or the pharmaceutically acceptable salt thereof, wherein 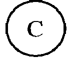 is benzene ring, preferably, R₃ is methyl, C1-C6 alkyl or alkoxy; C3-C6 cycloalkyl; C2-C6 alkenyl; C2-C6 alkynyl; halogen: including -F, Cl, Br, -CH₂Cl, -CH₂Br, -CHCl₂, - CCl₃, -CH₂F, -CHF₂, -CF₃; -CN, hydroxyl, amino or alkyl substituted amino.

3. The compound according to claim 1, wherein 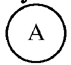 has a structure selected from the group consisting of 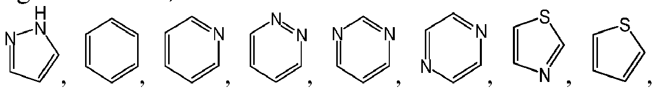 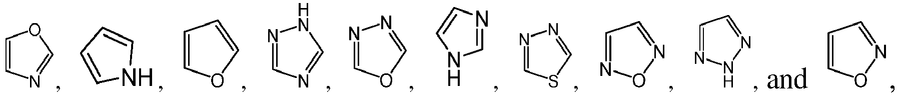 wherein the bonding position of the ring can be N or C.

4. The compound according to claim 1, or the optical isomer, hydrate, solvate thereof, or the pharmaceutically acceptable salt thereof, wherein 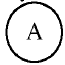 ring has a substitutent as shown in the following formula IV: 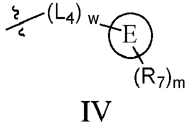 wherein, w is 0, 1, 2, 3, 4, 5, 6;
each L₄ is independently selected from the group consisting of substituted or unsubstituted C1-C4 alkylene, -S-, -O-, NR_{f}, -S(O)-, and -S(O)₂-; preferably substituted or unsubstituted C1-C4 alkylene; wherein, a hydrogen on a carbon atom of the substituted or unsubstituted C1-C4 alkylene can be each independently substituted by deuterium, provided that a structure formed by each L₄ is chemically stable; 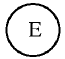 is selected from the group consisting of substituted or unsubstituted C3-C10 cycloalkyl, substituted or unsubstituted 3-10 membered heterocyclyl having 1-3 heteroatoms selected from B, P, N, S and O; preferably, 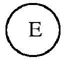 is 3-8 membered nitrogen-containing heterocyclyl, or substituted or unsubstituted 4-10 membered cyclic amido, wherein a hydrogen on the ring-forming carbon atom of 3-8 membered nitrogen-containing heterocyclyl, substituted or unsubstituted 4-10 membered cyclic amido can be independently substituted by deuterium;
each R₇ is independently selected from the group consisting of substituted or unsubstituted C1-C6 alkyl, -CN, hydroxy, amino, carboxyl, -OR_{g}, -N(R_{g})₂, -CO-NH-SO₂-R_{g}, -NH-SO₂-R_{g}, -SO₂-NH-CO-R_{g}, -CO₂R_{g}, -CON(R_{g})₂, -CONHCOR_{g}, NR_{g}-CO-N(R_{g})₂, and -NRg-SO₂-N(Rg)₂;R_{f} and R_{g} are defiend as above; wherein a hydrogen on a carbon atom of Rf and Rg can be independently substituted by deuterium; wherein, a substituent is selected from the group consisting of halogen, hydroxyl, carboxyl, cyano, and C1- C6 alkoxy.

5. The compound of claim 1, wherein 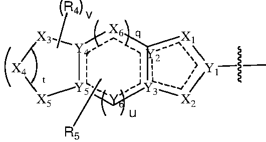 is selected from the group consisting of: 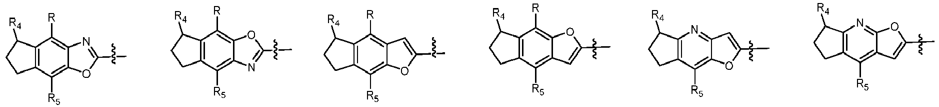 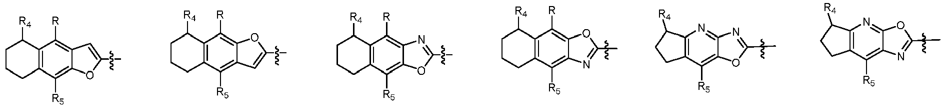 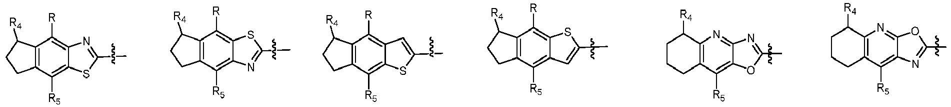 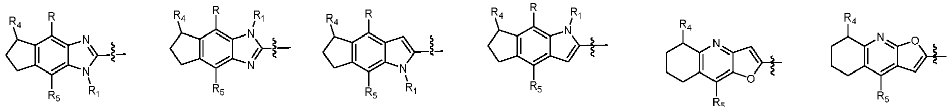 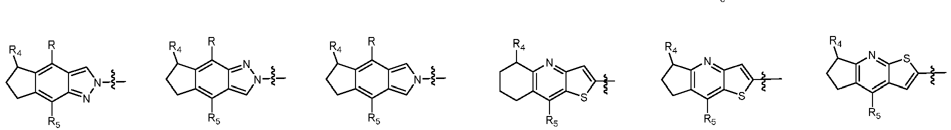 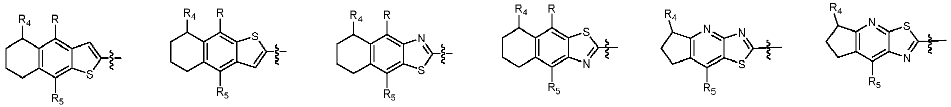 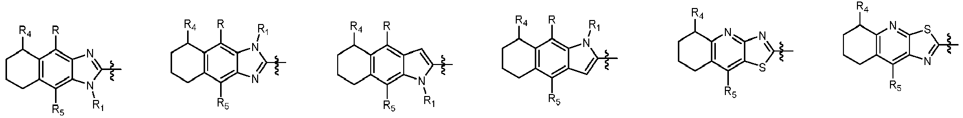 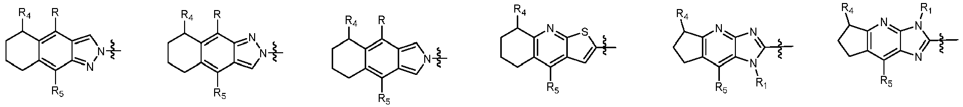 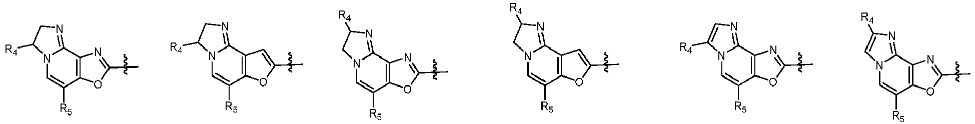 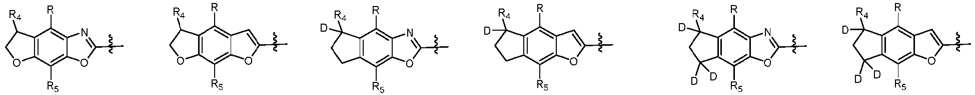 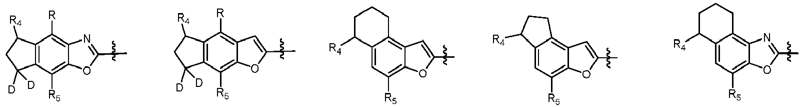 and

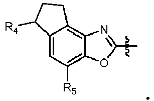

6. The compound according to claim 1, or the optical isomer, hydrate, solvate thereof, or the pharmaceutically acceptable salt thereof, wherein R₄ is selected from the group consisting of NRdRe; Rd and Re are each independently selected from the group consisting of H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 3-10 membered cycloalkyl, or substituted or unsubstituted 3-10 membered heterocyclyl having 1-3 heteroatoms selected from N, S and O; in another preferred embodiment, a substituent herein is carboxyl, hydroxyl, 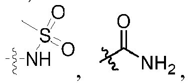 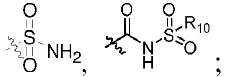 R₁₀ is substituted or unsubstituted C₁-C₆ alkyl, substituted or unsubstituted C₃-C₁₀ cycloalkyl, preferably, R₁₀ is methyl, isopropyl, cyclopropyl and 1-methylcyclopropyl, wherein a hydrogen on a carbon atom of Rd, Re and R₁₀ can be independently substituted by deuterium.

7. The compound of claim 1, or the optical isomer, hydrate, solvate thereof, or the pharmaceutically acceptable salt thereof, wherein the compound is selected from the following table;

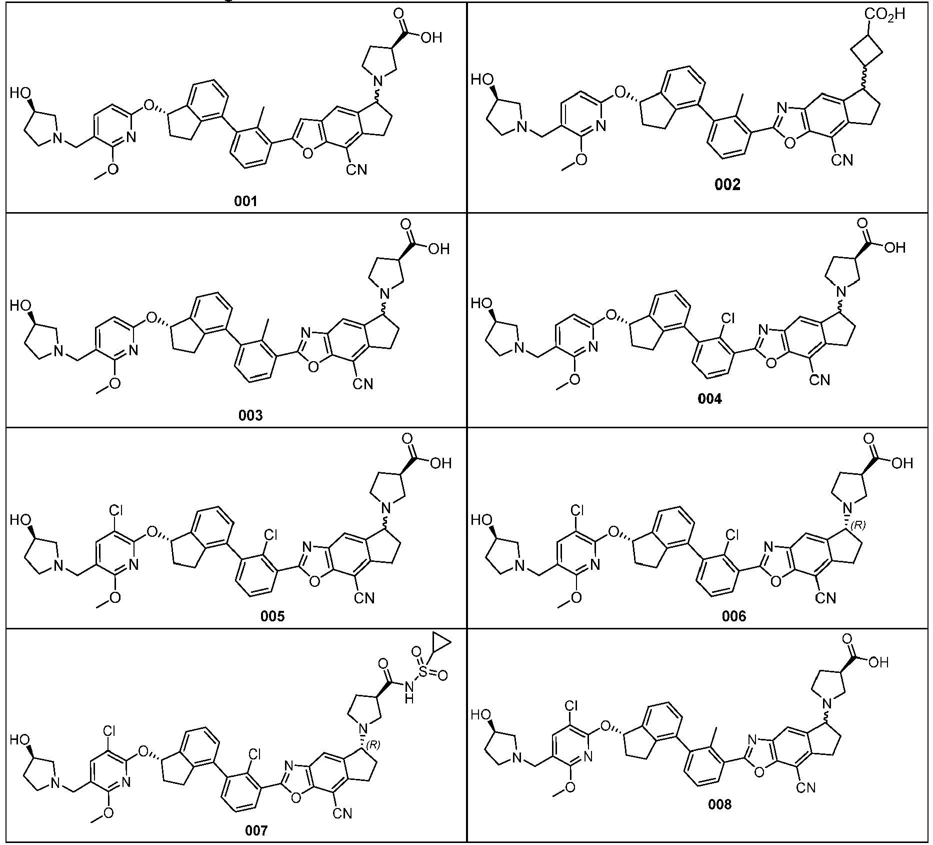

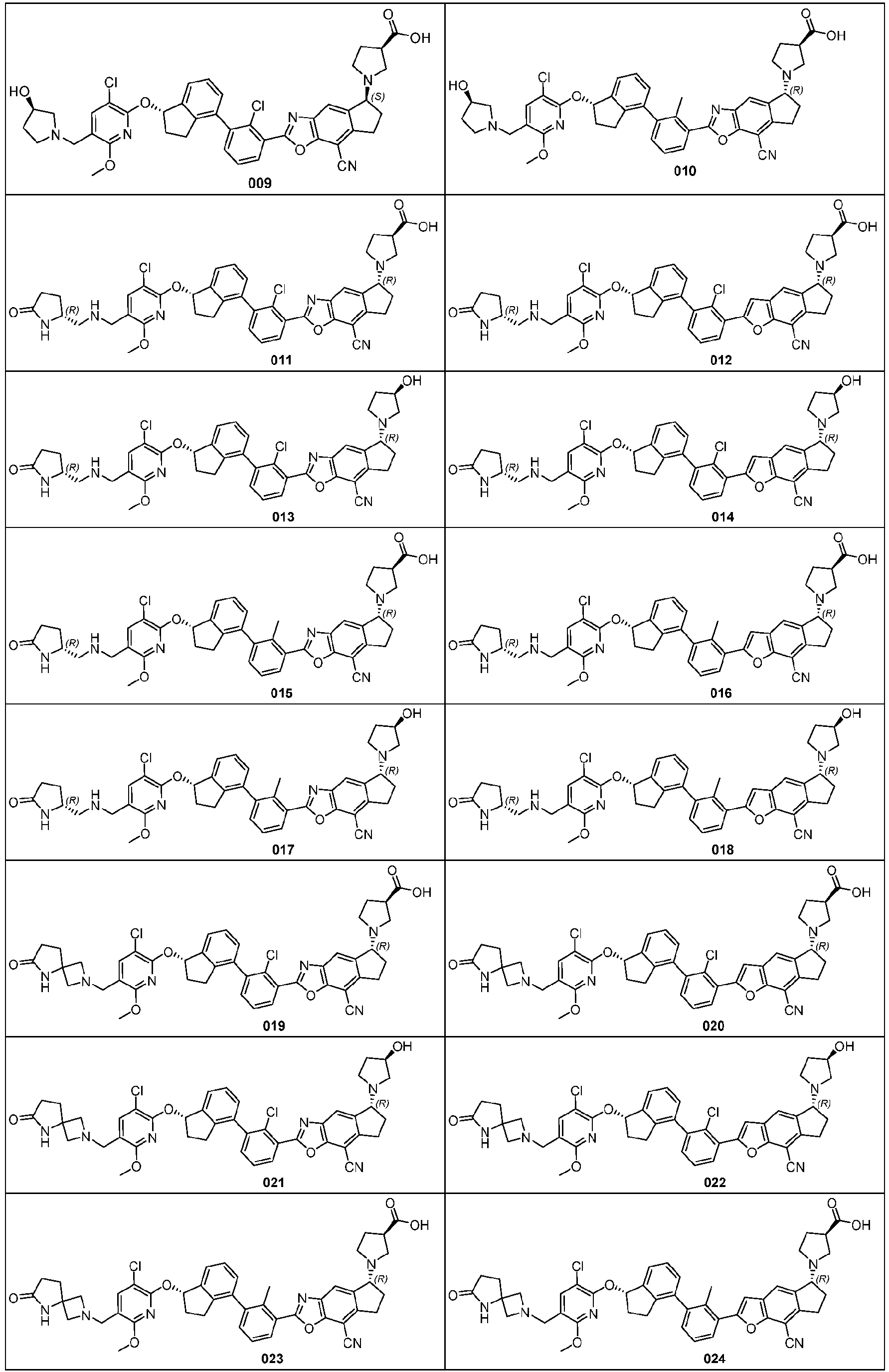

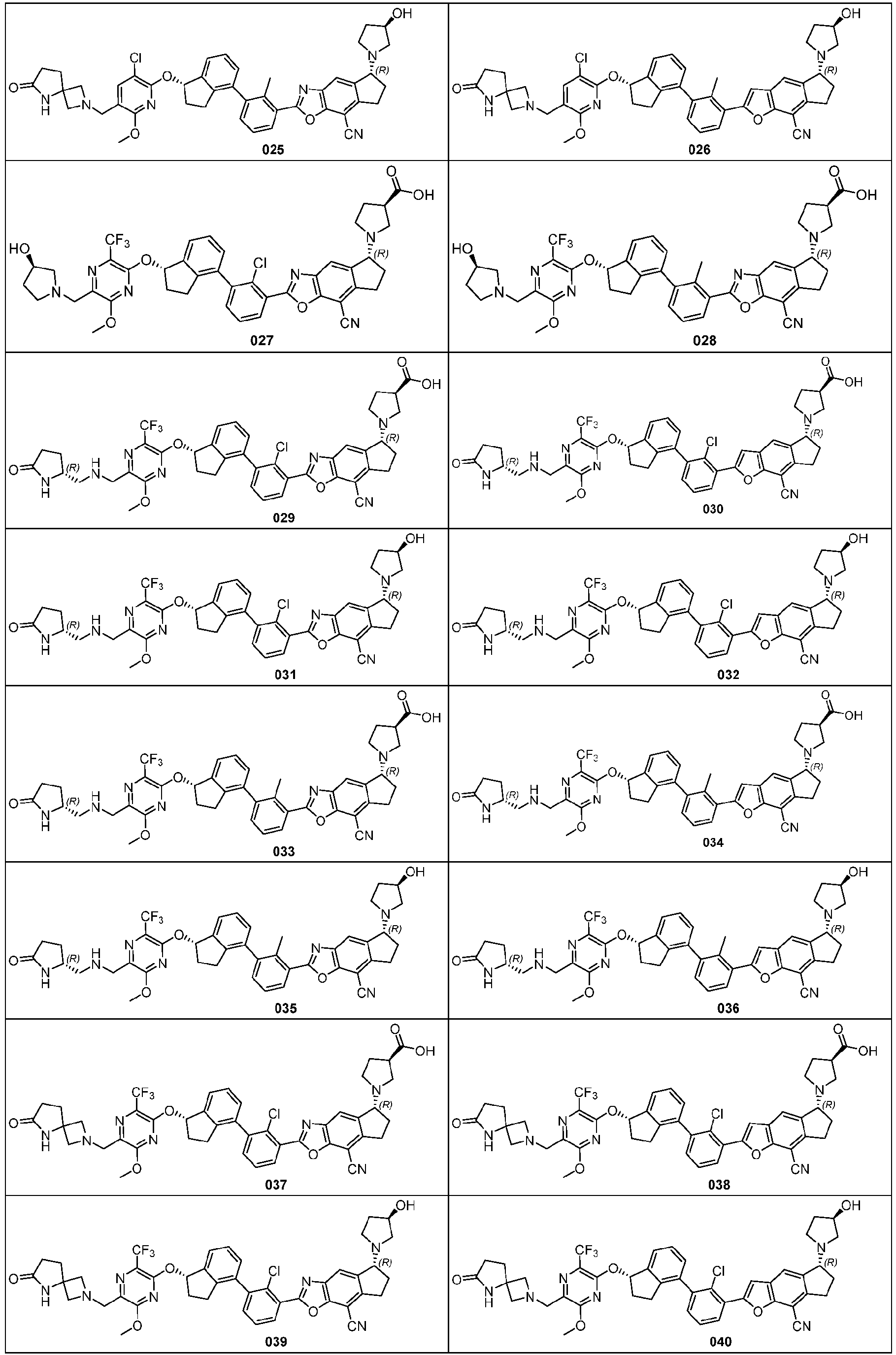

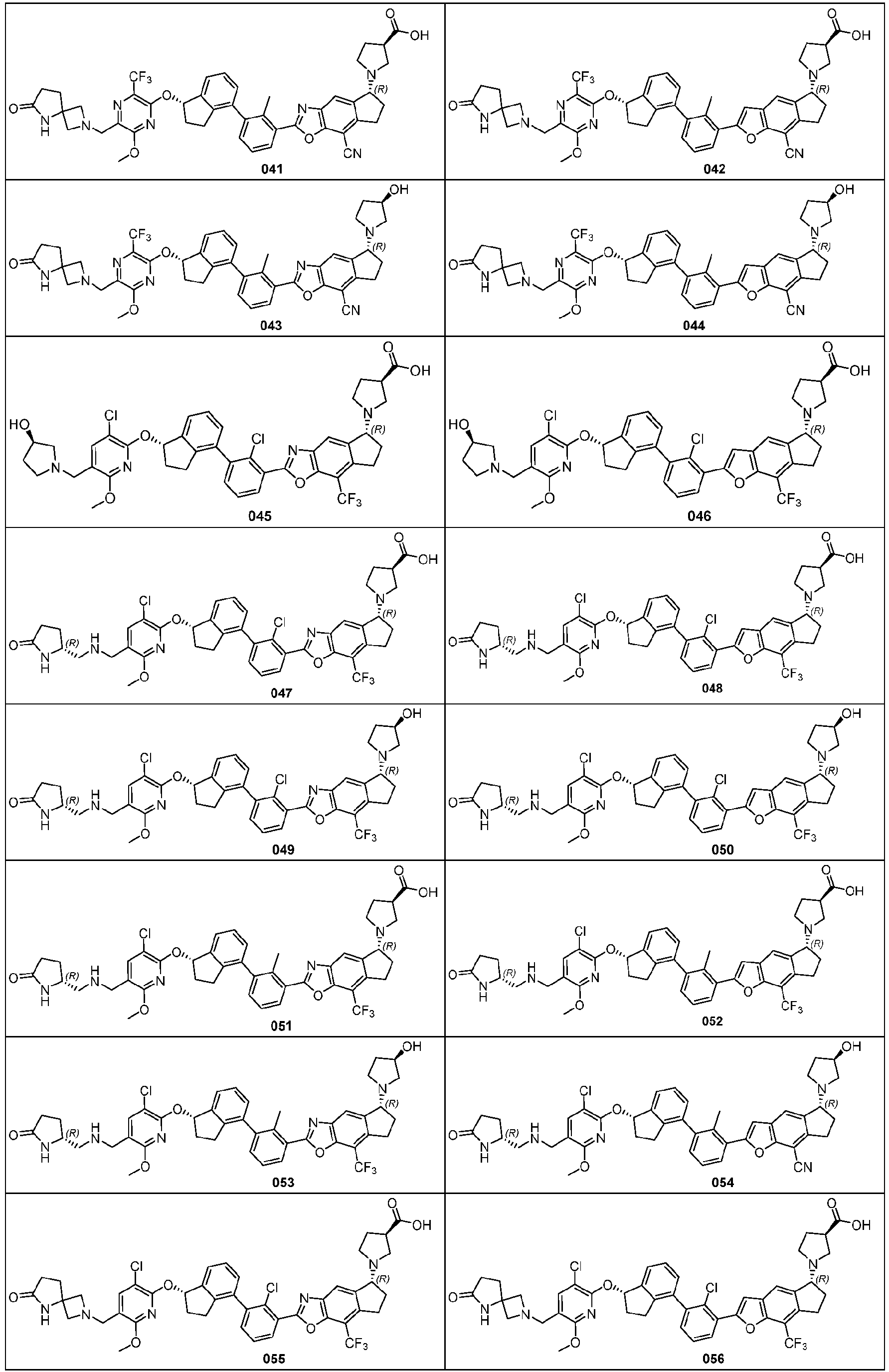

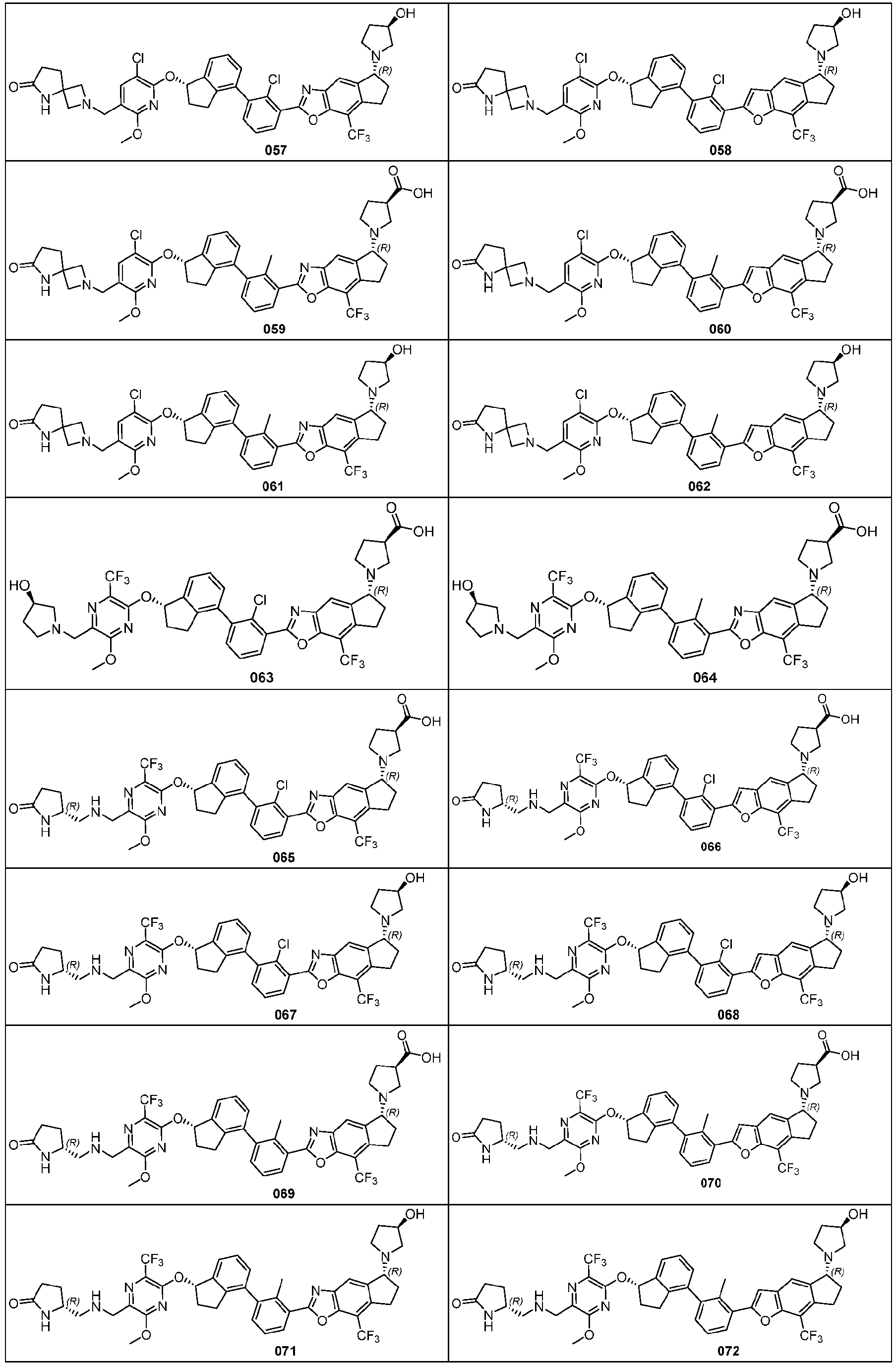

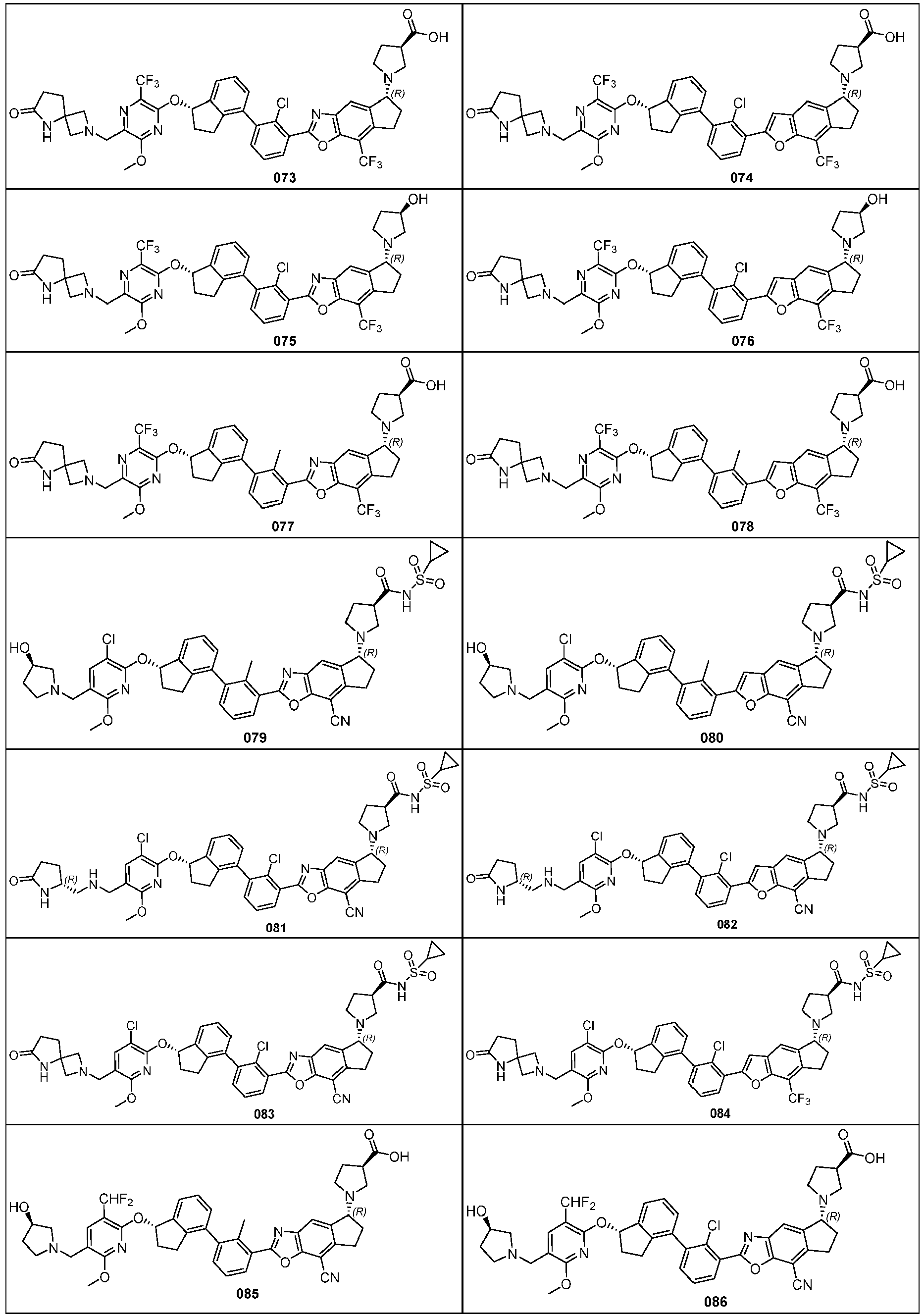

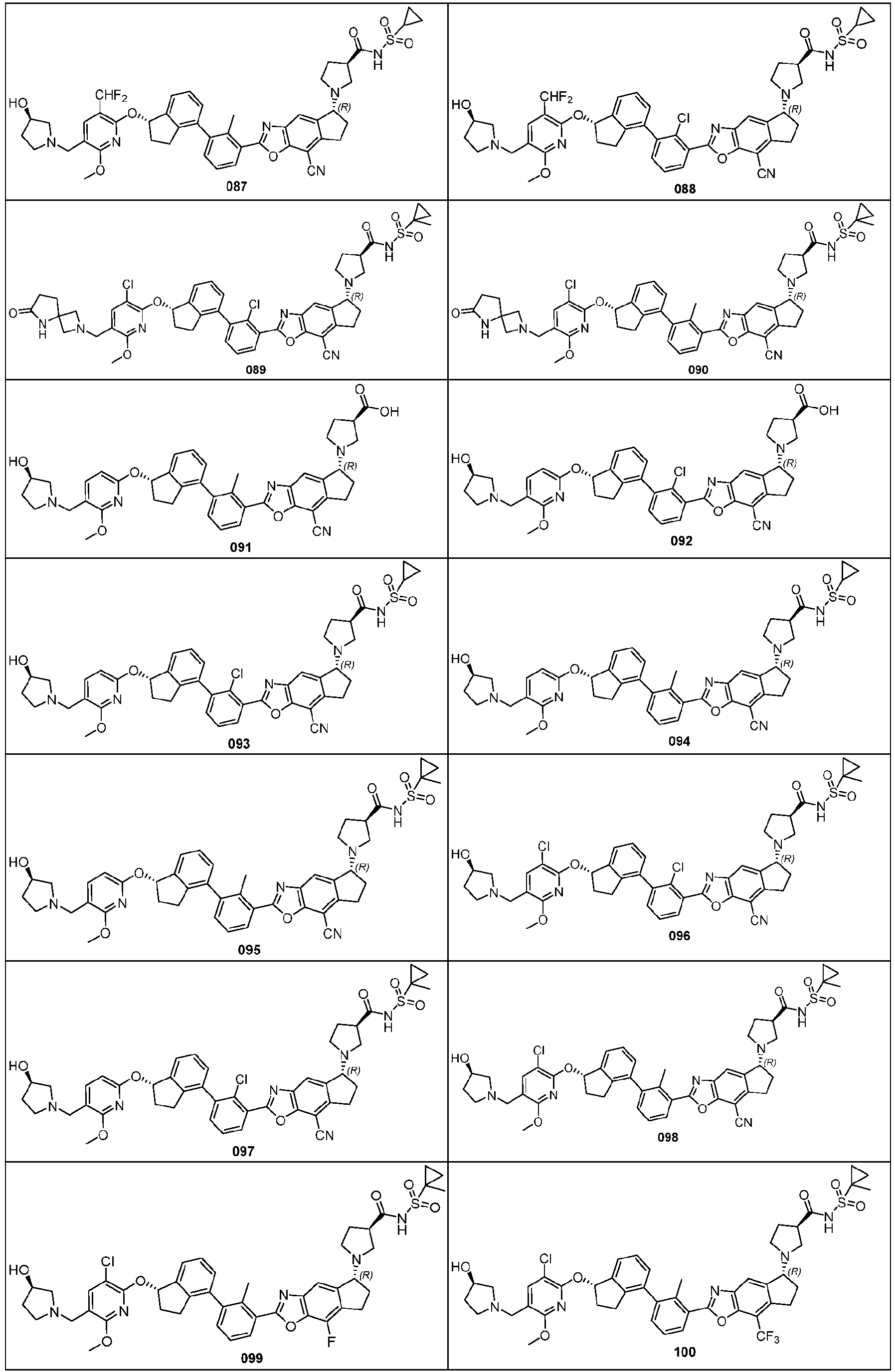

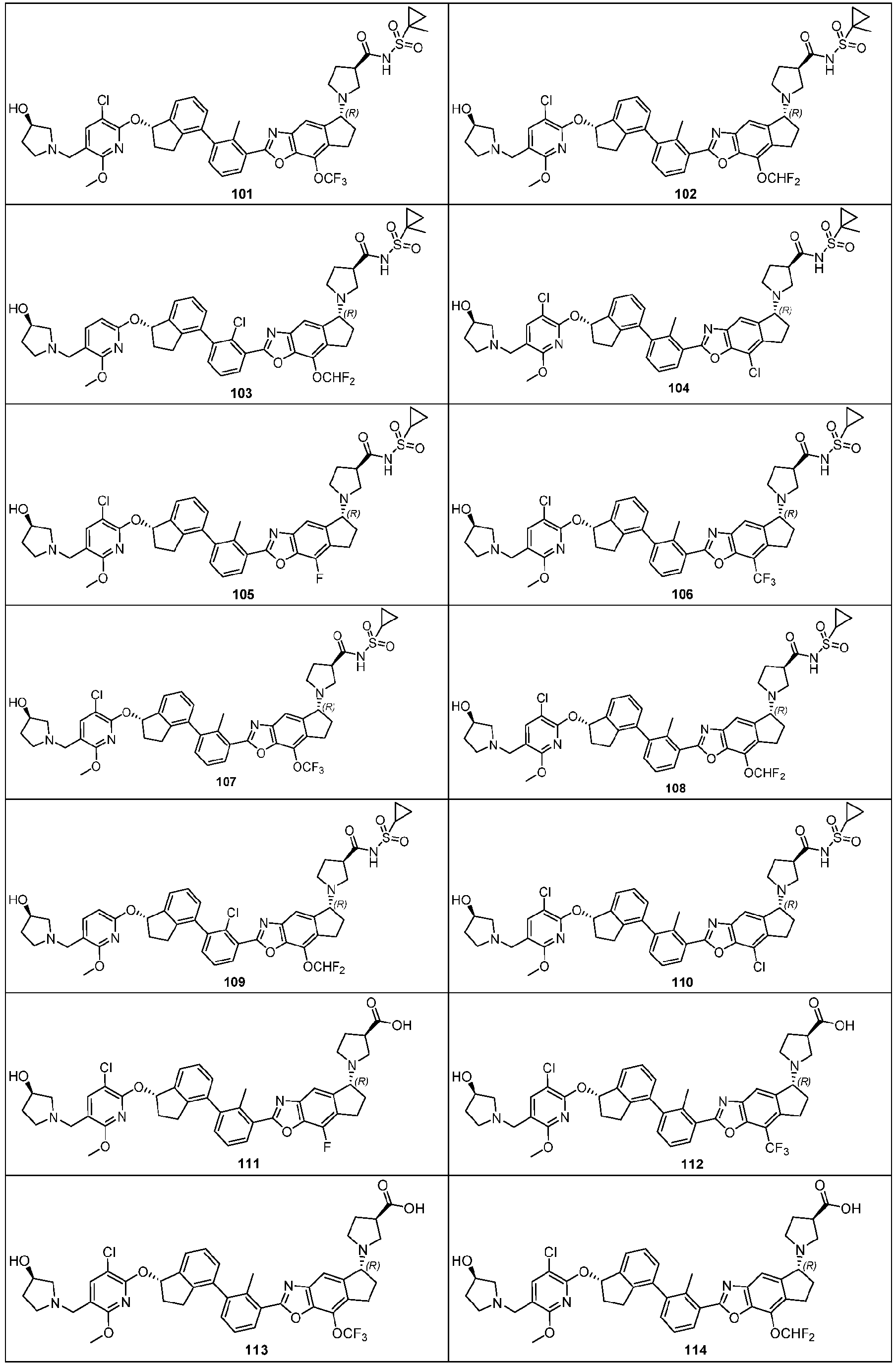

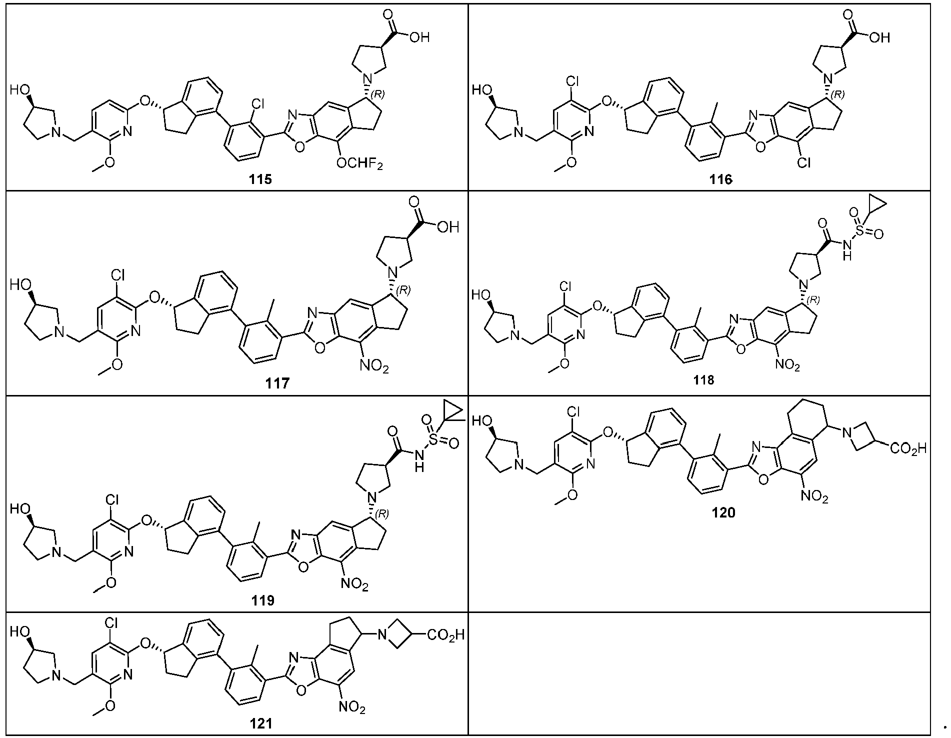

8. A preparation method of a compound of formula I according to claim 1, wherein the method comprises steps selected from the steps shown in Synthesis Scheme 1 or 2: 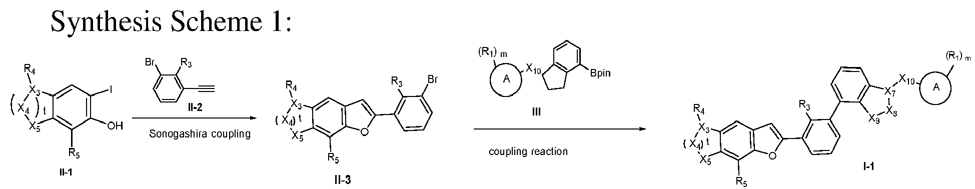
(a) subjecting intermediates II-1 and II-2 as raw materials to Sonogashira coupling reaction catalyzed by palladium catalyst to obtain intermediate II-3;
(b) subjecting II-3 and III-1 as raw materials to a coupling reaction (such as Suzuki, Buchwald, etc.) in the presence of palladium catalyst and ligand to obtain intermediate 1-1;
preferably, the preparation method of intermediate II-1 is as follows: 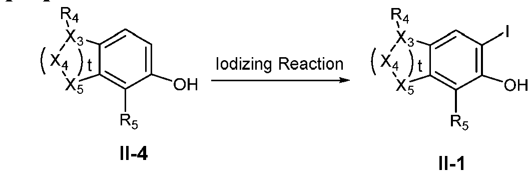 subjecting II-4 as raw material to a halogenation reaction catalyzed by Lewis acid to obtain intermediate II-1;
preferably, the preparation method of intermediate III-1 is as follows: 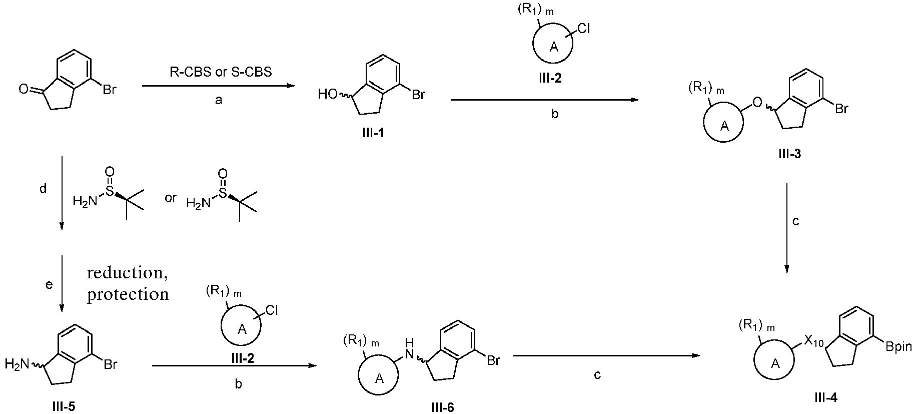
(a) reacting compound 4-bromoindanone as a raw material under the action of chiral auxiliary (such as R/S-CBS) and reducing agent (such as borane) to form chiral alcohol **1-2;**
(b) subjecting **III-1** (or **III-5**) and **III-2** as raw materials to an affinity substitution reaction under basic condition to obtain intermediate **III-3** (or **III-6**);
(c) subjecting **III-3** (or **III-6**) and bis(pinacolato)diboron as raw materials to a boronation reaction under the presence of palladium catalyst and ligand to obtain intermediate **III-1;**
(d), (e) reacting compound 4-bromoindanone and R/S-tert-butylsulfonimide as raw materials under the action of Lwesis acid (such as ethyl tetratitanate) and reducing agent (such as lithium aluminum tetrahydride, sodium borohydride, etc.) to form a protected chiral amino compound, and then the protective group is removed under acidic condition to obtain a chiral amino compound **III-5;** 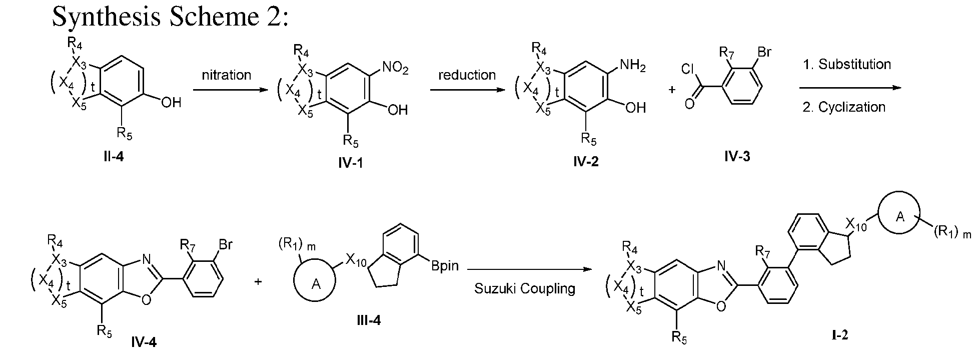
(a) reacting compound **II-4** as a raw material with a nitrifying agent (such as concentrated sulfuric acid/NaNO₃, concentrated sulfuric acid/fuming nitric acid, etc.) to form intermediate **IV-1;**
(b) subjecting **IV-1** as a raw material to a reduction reaction under reducing condition (Pd-C/H₂; zinc powder/ammonium chloride; iron powder/acetic acid etc.) to form intermediate **IV-2;**
(c) subjecting **IV-2** and **IV-3** as raw materials to an affinity substitution reaction under alkaline conditionan to obtain amide intermediate; and then subjecting to a cyclization reaction in the presence of suitable dehydration reagent (such as PPh₃/DDQ) to obtain an intermediate **IV-4;**
(d) subjecting **IV-4** and **IV-5** as raw materials to a coupling reaction under the conditions of catalyst and ligand to form target product **1-2;**
above X₁-X₁₀, R₁-R₁₀, t, and m are defined as above.

9. A pharmaceutical composition comprising (1) the compound according to claim 1, the stereoisomer or tautomer thereof, or the pharmaceutically acceptable salt, hydrate or solvate thereof; and (2) a pharmaceutically acceptable carrier.

10. Use of the compound according to claim 1 or the stereoisomer or tautomer thereof or the pharmaceutically acceptable salt, hydrate or solvate thereof, or the pharmaceutical composition according to claim 8, for the preparation of a pharmaceutical composition for preventing and/or treating diseases related to the activity or expression of PD-1/PD-L1.

11. The use according to claim 10, wherein the pharmaceutical composition is used to treat a disease selected from the group consisting of cancer, infectious disease, and autoimmune disease.

12. The use according to claim 10, wherein the cancer is selected from the group consisting of pancreatic cancer, bladder cancer, colorectal cancer, breast cancer, prostate cancer, kidney cancer, hepatocellular carcinoma, lung cancer, ovary cancer, cervical cancer, stomach cancer, esophageal cancer, melanoma, neuroendocrine cancer, central nervous system cancer, brain cancer, bone cancer, soft tissue sarcoma, non-small cell lung cancer, small cell lung cancer or colon cancer, skin cancer, lung cancer, urinary system tumor, blood tumor, glioma, digestive system tumor, reproductive system tumor, lymphoma, nervous system tumor, brain tumor, head and neck cancer.

13. The use according to claim 10, wherein the infectious disease is selected from bacterial infection and viral infection.

14. The use according to claim 10, wherein the autoimmune disease is selected from the group consisting of organ-specific autoimmune disease and systemic autoimmune disease.

15. The use according to claim 10, wherein the pharmaceutical composition further comprises at least one therapeutic agent selected from the group consisting of nivolumab, pembrolizumab, atezolizumab and ipilimumab.
